# EUROPEAN PATENT APPLICATION

(11) **EP 4 666 944 A1**
(43) Date of publication of application: **24.12.2025**
(21) Application number: 25183860.3
(22) Date of filing: 18.06.2025
(51) Int. Cl.: A61B 5/15, A61B 5/151

(54) **BLOOD COLLECTION PEN**

(30) Priority: 19.06.2024 CN 202410795938
(71) Applicant: Tianjin Huahong Technology Co., Ltd., Tianjin 300308 (CN)
(72) Inventor: YANG, Hao, Dongli District, Tianjin (CN); ZHANG, Libo, Dongli District, Tianjin (CN)
(74) Representative: Bandpay & Greuter

(57) **Abstract**

The present disclosure relates to a blood collection pen, including: a guide sleeve (5); an inner core (2), a front end of which is provided with a needle mounting mechanism for detachably installing a blood collection needle, and a rear end of which passes through the guide sleeve; a needle retraction rod (3) provided through the inner core in an axial direction, a front end of the needle retraction rod being adapted for passing through an inner core hole at a front end of the inner core; wherein: the inn core includes an elastic structure which is adapted for abut against an inner wall of the guide sleeve in the axial direction; and the elastic structure is adapted to abut against the needle retraction rod in a transverse direction to prevent a part of the elastic structure that abuts against the inner wall of the guide sleeve from elastically deforming or moving laterally inward in a case that the blood collection needle is not installed at the front end of the inner core.

## Description

### TECHNICAL FIELD

An embodiment of the present disclosure relates to the field of medical instruments, in particular to a blood collection pen.

### BACKGROUND

A traditional blood collection pen is used as follows: unscrewing a pen cap, putting a blood collection needle into a needle seat, unscrewing the needle cap, installing the pen cap, and loading the blood collection pen and launching for blood collection; unscrewing the pen cap, piercing the needle tip of the blood collection needle into the needle cap, discarding the blood collection needle and installing the pen cap. Not only the use steps are complicated, but also in the process of unscrewing the needle cap to install the pen cap and unscrewing the pen cap to discard the blood collection needle, the needle tip of the blood collection needle is exposed and unprotected, which may lead to the risk of injury.

Moreover, there is a potential risk in a blood collection pen in which the blood collection needle is directly installed from the pen cap into the needle seat. That is, when the blood collection needle is not installed in place, the user may be harmed if the blood collection pen is loaded and launched to eject the blood collection needle from the pen cap hole.

### SUMMARY

The present disclosure is proposed to alleviate or solve at least one aspect or at least one point of the above problems.

An embodiment of the present disclosure provides a blood collection pen, including:
a guide sleeve;
an inner core, a front end of which is provided with a needle mounting mechanism for detachably installing a blood collection needle, and a rear end of which passes through the guide sleeve;
a needle retraction rod provided through the inner core in an axial direction, a front end of the needle retraction rod being adapted for passing through an inner core hole at a front end of the inner core,
wherein:
the inn core includes an elastic structure which is adapted for abut against an inner wall of the guide sleeve in the axial direction; and
the elastic structure is adapted to abut against the needle retraction rod in a transverse direction to prevent a part of the elastic structure that abuts against the inner wall of the guide sleeve from elastically deforming or moving laterally inward in a case that the blood collection needle is not installed at the front end of the inner core.

### BRIEF DESCRIPTION OF DRAWINGS

FIG. 1 is an explosion schematic diagram of a blood collection pen according to an exemplary embodiment of the present disclosure;
FIGS. 2 and 3 are perspective schematic views of the pen cap in FIG. 1;
FIGS. 4 to 6 (a) and 6(b) are three-dimensional schematic views of the inner core in FIG. 1 from different perspectives;
FIGS. 7(a) and 7(b) are perspective schematic views of the needle retraction rod in FIG. 1;
FIGS. 8 and 9 are perspective views of the blocking member in FIG. 1 from different perspectives;
FIGS. 10 to 13 are perspective views of the guide sleeve in FIG. 1 from different perspectives;
FIGS. 14 to 16 are perspective views of the main body in FIG. 1 from different perspectives;
FIGS. 17 to 18 are perspective views of the launching key in FIG. 1 from different perspectives;
FIGS. 19 to 20 are perspective views of the tail cap inner core in FIG. 1 from different perspectives;
FIGS. 21 to 22 are perspective views of the tail cap in FIG. 1 from different perspectives;
FIGS. 23 to 25 are perspective views of the shift adjustment key in FIG. 1 from different perspectives;
FIGS. 26 to 27 are perspective views of the needle withdrawing key in FIG. 1 from different perspectives;
FIG. 28 is a schematic perspective view of the blood collection needle in FIG. 1;
FIGS. 29 to 31 are state diagrams when the blood collection pen is not installed with a needle and is not loaded;
FIGS. 32 to 34 are state diagrams of pulling the tail cap when the blood collection pen is not installed with a needle;
FIGS. 35 to 38 show the process of installing the blood collection needle into the blood collection pen and the state that the blood collection pen is not loaded after the blood collection needle is installed;
FIGS. 39 to 41 are diagrams showing the loading state of the blood collection pen after the needle is installed;
FIGS. 42 to 44 are state diagrams of loading after the blood collection pen is installed with the needle and loaded and the needle cap is removed;
FIGS. 45 to 47 are state diagrams when the blood collection needle is launched to the front end after the blood collection pen is launched;
FIGS. 48 to 49 are diagrams showing the recovery to the natural state after the launching process of the blood collection needle is completed;
FIGS. 50 to 51 are state diagrams of pressing the needle withdrawing key until the needle tip is exposed from the pen cap;
FIG. 52 to FIG. 53 are state diagrams of pressing the needle withdrawing key until the needle tip of the blood collection needle is pierced into the needle cap;
FIG. 54 to FIG. 55 are state diagrams of the blood collection pen after the needle is withdrawn;
FIG. 56 (a) to FIG. 56(d) are the relative position state diagrams of the inner core and the needle retraction rod respectively when the needle is not installed, installed, loaded and launched.

### Reference signs:

1, Pen cap;
101, Pen cap protrusion, 102, Pen cap hole;
2, Inner core;
201, Mounting base, 202, Elastic arm structure, 203, Inner core locating rib, 204, Hollowed-out part, 205, Elastic arm, 206, Elastic arm end part, 207, Elastic structure, 208, First axial limit matching portion, 209, Inner core groove, 210, Inner core distal end, 211, 211, Lateral limit part, 212, Inner core holes, 213, Locating rib end part, 214, Inner core cavity, 215, First blocking part, 216, Inner core abutting part, 217, Inner core tail end face, 218, Inner core bottom face, 219, protrusion;
3, Needle retraction rod;
301, Needle retraction rod top end, 302, Avoidance space, 303, Needle -retraction - rod locating rib, 304, First blocking matching portion, 305, Second blocking matching portion, 306, Needle retraction rod rear end, 307, Concave side wall;
4, Blocking member;
401, Blocking member hole, 402, Blocking member groove, 403, Blocking member front end face, 404, Blocking member abutting face;
5, Guide sleeve;
501, Guide sleeve stepped face, 502, Guide sleeve wall face, 503, Clamping groove, 504, Locating member, 505, Locating face, 506, Guide sleeve tail end wall face, 507, Guide sleeve hole, 508, Guide sleeve groove, 509, Groove bottom end face, 510, Guide sleeve inner wall, 511, Guide sleeve abutting face, 512, Guide sleeve locating groove, 513, First axial limit part, 514, Protruding inclined plane, 515, First protrusion;
6, Main body;
601, Main body hole, 602, Circumferential rib, 603, Main body locating rib, 604, Main body locating groove, 605, Main body rear end face, 606, Main body key hole, 607, Main body sight hole;
7, Launching key;
701, Launching rib, 702, Snap lock;
8, Tail cap inner core;
801, Tail cap inner core Locating groove, 802, Locating groove end face, 803, Elastic member, 804, Spiral groove, 805, Tail cap inner core abutting end face, 806, Tail cap inner core hole, 807, Hollow structure, 808, Tail cap inner core rear end face;
9, Tail cap;
901, Tail cap front end, 902, Tail cap stepped face, 903, Viewport, 904, Tail cap locating rib, 905, Inner wall groove, 906, Tail cap rib, 907, Tail cap rib end face, 908, Tail cap cavity, 909, Tail cap rear end face;
10, Shift adjustment key;
1001, Adjustment key rib, 1002, Adjustment key groove, 1003, Indicator number, 1004, Shift adjustment key stepped face, 1005, Boss structure, 1006, Rear end stepped face, 1007, Adjustment key inner wall, 1008, Adjustment key hole;
11, Needle withdrawing key;
1101, Needle withdrawing key rear end, 1102, Front end central structure, 1103, Needle withdrawing key elastic arm, 1104, Needle withdrawing key front end face, 1105, Needle withdrawing key step, 1106, Needle withdrawing key bottom end;
12, First spring;
13, Second spring;
14, Blood collection needle;
1401, Needle cap, 1402, Needle body, 1403, Blood collection needle stepped face, 1404, Blood collection needle bottom face.

### DESCRIPTION OF EMBODIMENTS

The technical solution in the embodiment of the present disclosure will be clearly and completely described below with reference to the attached drawings. Obviously, the described embodiment is only part of the embodiments, rather than all of the embodiments of the present disclosure. Based on the embodiments in the present disclosure, all other embodiments obtained by those skilled in the art based on this application shall belong to the protection scope of this disclosure.

Referring to the attached drawings, the present disclosure provides a blood collection pen, including:
a guide sleeve 5;
an inner core 2, a front end of which is provided with a needle mounting mechanism for detachably installing a blood collection needle, and a rear end of which is adapted to passes through the guide sleeve;
a needle retraction rod 3 provided through the inner core in an axial direction, a front end of the needle retraction rod being adapted for passing through an inner core hole at a front end of the inner core,
wherein
the inn core 2 includes an elastic structure 207 which is adapted for abut against an inner wall of the guide sleeve in the axial direction; and
the elastic structure is adapted to abut against the needle retraction rod in a transverse direction to prevent a part of the elastic structure that abuts against the inner wall of the guide sleeve from elastically deforming or moving laterally inward in a case that the blood collection needle is not installed or not properly installed at the front end of the inner core.

Hereinafter, the technical solution of the present disclosure will be described in more detail with reference to the attached drawings.

The "proximal end", "front end", "head part" and "front part" mentioned in this disclosure refer to the end close to the needle outlet of the blood collection needle (or the end where the blood collection needle is installed after the blood collection pen is assembled); the "distal end", "rear end", "rear part" and "tail part" are the end far away from the needle outlet; "inner" refers to the direction towards the center of the cavity, and "outer" refers to the direction away from the center of the cavity.

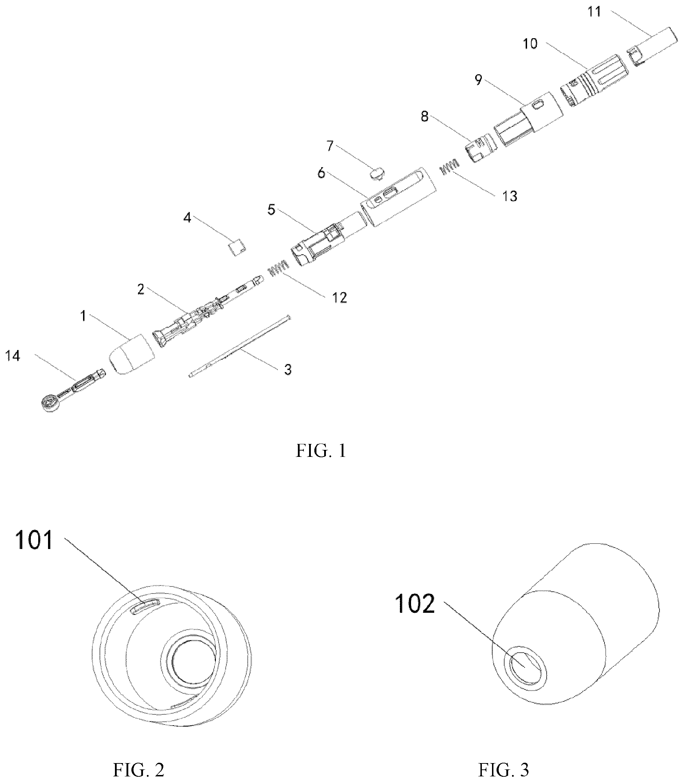

According to an exemplary embodiment of the present disclosure, as shown in FIG. 1, a blood collection pen includes a pen cap 1, an inner core 2, a needle retraction rod 3, a blocking member 4, a guide sleeve 5, a main body 6, a launching key 7, a tail cap inner core 8, a tail cap 9, a shift adjustment key 10 and a needle withdrawing key 11.

According to an exemplary embodiment of the present disclosure, as shown in FIGS. 2 and 3, the specific structure of the pen cap 1 is shown. The inner wall of the pen cap 2 is provided with a pen cap protrusion 101 protruding radially inward, and one end of the needle outlet is provided with a pen cap hole 102.

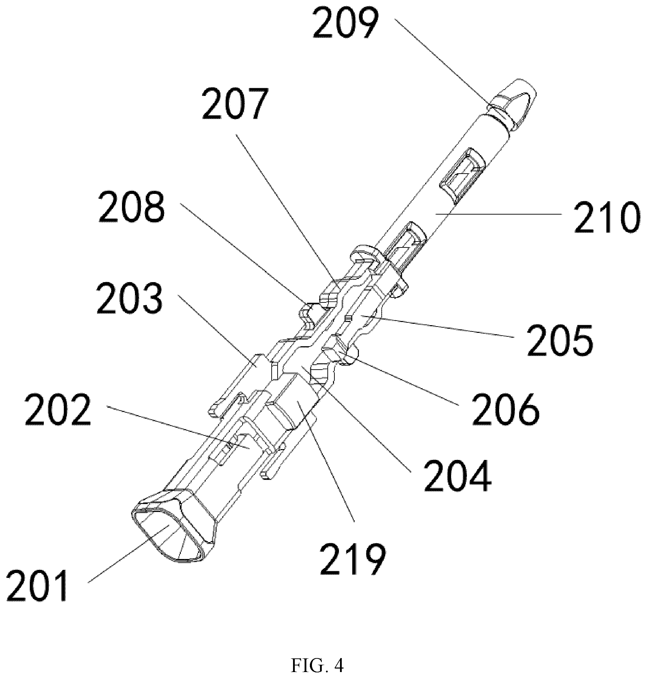

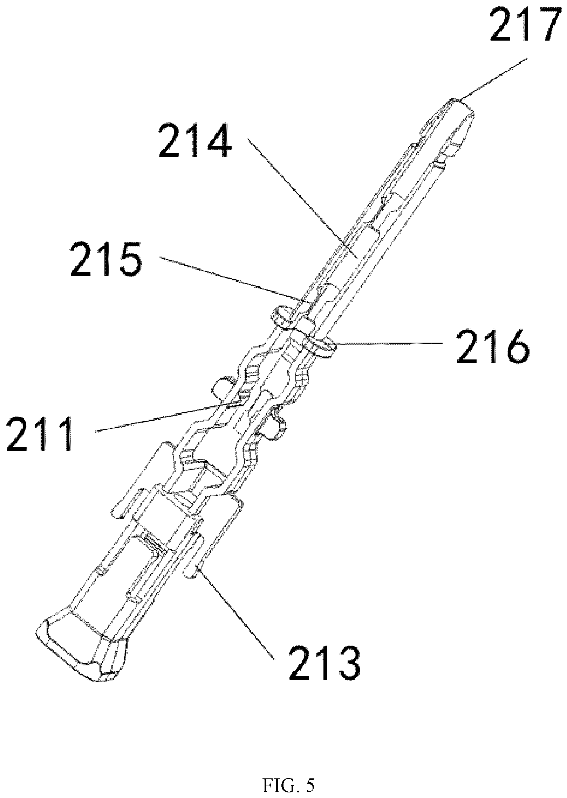

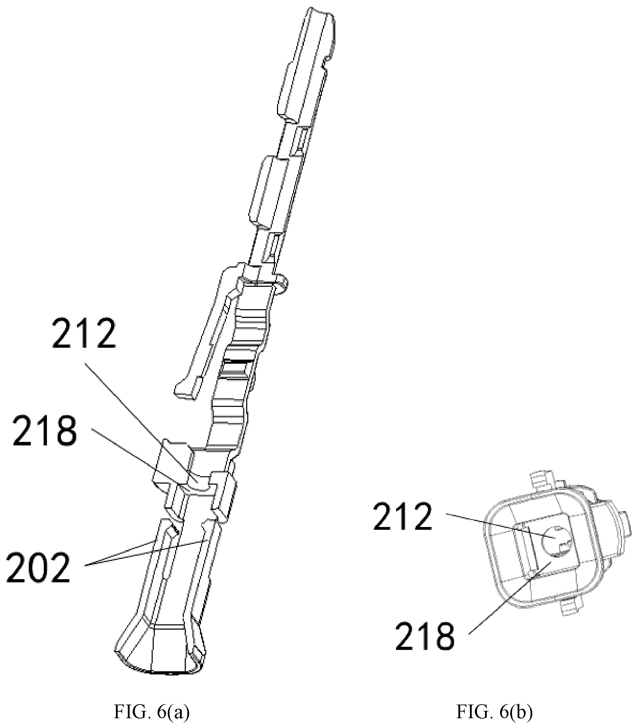

According to an exemplary embodiment of the present disclosure, as shown in FIGS. 4 to 6(a) and 6(b), the specific structure of the inner core 2 is shown.

The inner core 2 has an inner core cavity 214, and its front end has a mounting base 201 for mounting a blood collection needle. The inner core 2 is provided with an elastic arm structure 202 extending from the proximal end to the distal end, and the end of the elastic arm structure 202 is used for clamping the blood collection needle stepped face 1403. The inner core 2 is provided with inner core locating ribs 203 that are symmetrically provided, and a hollow part 204 is provided in the inner core cavity. An elastic arm 205 is also provided on the inner core 2, and the end of the elastic arm 205 protrudes outward as an elastic arm end part 206, which is adapted for contacting with the launching key 7.

The inner core is provided with an elastic structure 207 extending in the axial direction, and optionally, the elastic structure 207 is two structures provided symmetrically in the radial direction. The inner core 2 on the same side as the inner core locating rib 203 is provided with a first axial limit matching portion 208. Optionally, the first axial limit matching portion 208 is a protrusion structure protruding outward. The first axial limit matching portion 208 is provided on the elastic structure 207.

The inner core distal end 210 has an inner core groove 209 provided in the circumferential direction.

The inner wall of the inner core is provided with symmetrically provided lateral limit parts 211. Optionally, the lateral limit parts 211 are protrusion structures protruding inward from the inner wall face, and the lateral limit parts 211 have inclined planes in the axial direction. Optionally, the number of lateral limit parts 211 may be one.

The inner wall of the inner core 2 is also provided with a first blocking part 215. Optionally, the first blocking part 215 is a protrusion structure protruding inward from the inner wall face. There may a plurality of first blocking parts 215 that are provided at intervals in the axial direction of the inner wall, and optionally, the first blocking parts 215 are provided on opposite sides of the inner wall of the inner core 2.

The outer wall of the inner core 2 is provided with an inner core abutting part 216 for abutting against one end of the first spring 12.

The inner core 2 has an inner core bottom face 218, and its side face is provided with a protrusion 219.

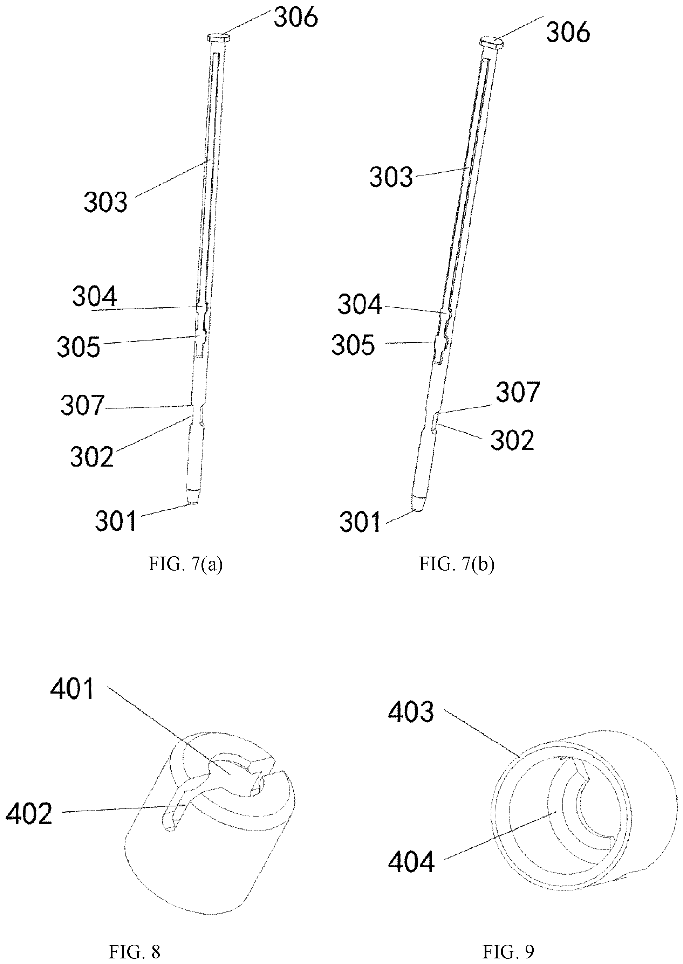

According to an exemplary embodiment of the present disclosure, as shown in FIGS. 7(a) and 7(b), the specific structure of the needle retraction rod 3 is shown.

The needle retraction rod 3 is provided with an avoidance space 302, which is configured as two grooves oppositely provided at the front end of the needle retraction rod 3. Optionally, there may be one avoidance space 302. There is a concave side wall 307 at the junction of the avoidance space 302 and the main part of the needle retraction rod 3. The needle retraction rod 3 is provided with an axially extending needle -retraction -rod locating rib 303, and the proximal end of the needle -retraction -rod locating rib 303 is provided with a second blocking matching portion 305 and a first blocking matching portion 304.

According to an exemplary embodiment of the present disclosure, as shown in FIGS. 8 and 9, the specific structure of the blocking member 4 is shown.

The proximal end of the blocking member 4 is an end with an opening, and the distal end of the blocking member 4 has a blocking member hole 401. The blocking member 4 extends from the distal end to the proximal end and is provided with blocking member grooves 402 provided symmetrically.

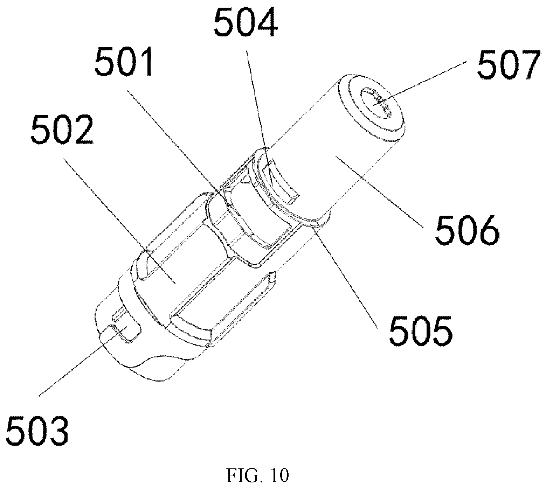

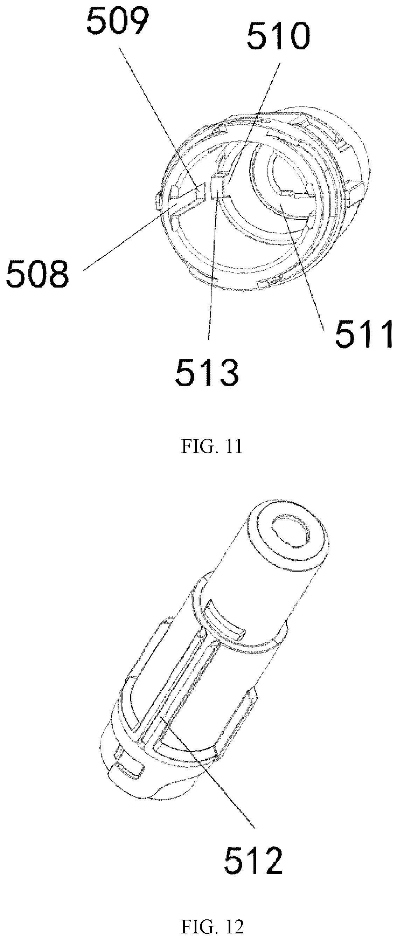

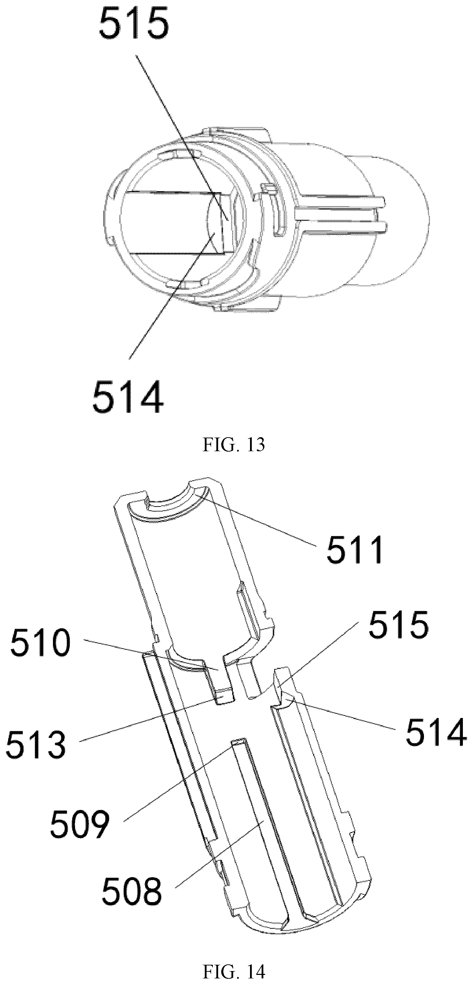

According to an exemplary embodiment of the present disclosure, as shown in FIGS. 10-13, the specific structure of the guide sleeve 5 is shown.

The proximal end of the guide sleeve 5 is provided with a clamping groove 503, and the distal end of the guide sleeve 5 is provided with a locating member 504 and a locating face 505. Optionally, the locating face 505 is a stepped face formed by the distal end of the guide sleeve 5 and the main part of the guide sleeve 5 based on the difference in circumferential radius. There is a certain distance between the locating member 504 and the locating face 505, and the distance is used to accommodate the circumferential rib 602 of the main body 6.

Optionally, the locating member 504 is closer to the distal end than the locating face 505.

Optionally, the locating member 504 is a radial protrusion provided on the outer wall of the guide sleeve 5.

The interior of the guide sleeve 5 is provided with a guide sleeve groove 508 for cooperating with the inner core locating rib 203. The outer wall face of the guide sleeve 5 is provided with a guide sleeve locating groove 512 for cooperating with the locating member 603 of the main body 6.

The guide sleeve 5 has a first axial limit part 513, and optionally, the first axial limit part 513 has an inclined plane structure.

The guide sleeve 5 has a first protrusion 515 protruding inward. The rear end of the first protrusion 515 has a stepped face 501, and the front end of the first protrusion 515 has a protruding inclined plane 514. The elastic arm end part 206 of the inner core 2 is adapted to slide over the protruding inclined plane 514 and is elastically deformed inward by the inner wall of the first protrusion 515 until the elastic arm end part 206 contacts the stepped face 501.

The front end of the guide sleeve 5 is provided with a clamping groove 503 for clamping and matching with the pen cap 1.

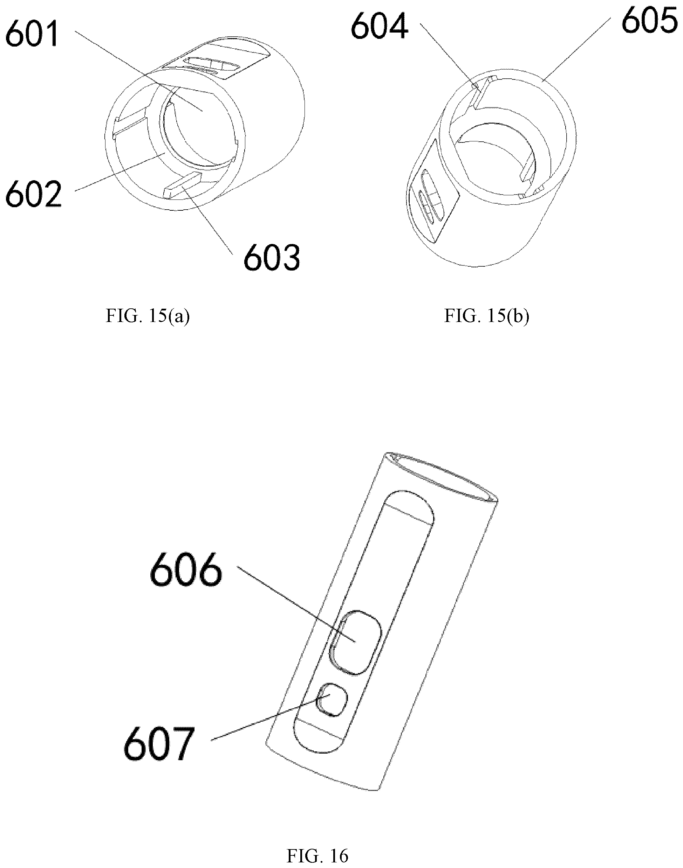

According to an exemplary embodiment of the present disclosure, as shown in FIGS. 15(a), 15(b) and 16, the specific structure of the main body 6 is shown.

Inside the main body 6, there are main body locating ribs 603 that axially extend and circumferential ribs 602 on the circumference. The rear end of the main body 6 is provided with a main body locating groove 604, and the side wall of the main body 6 is provided with a main body key hole 606 and a main body sight hole 607.

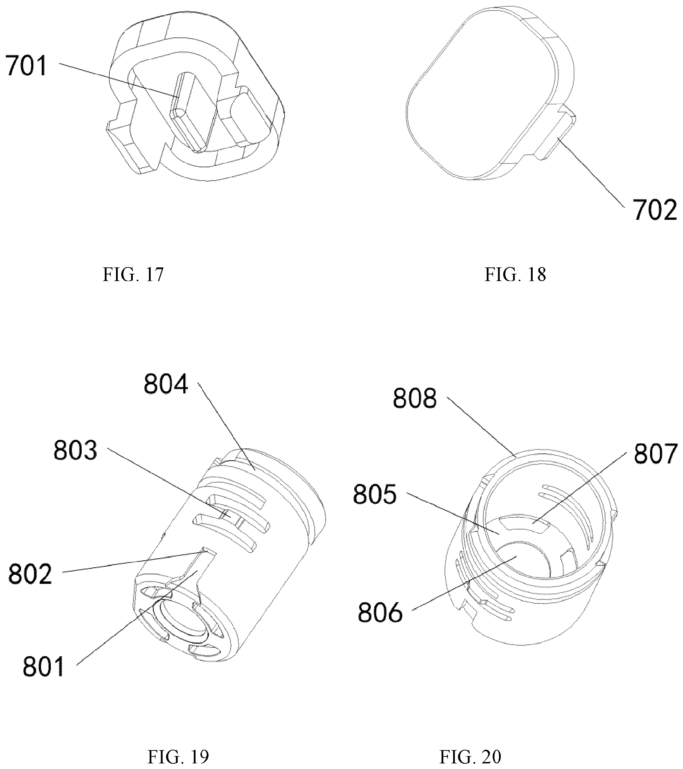

According to an exemplary embodiment of the present disclosure, as shown in FIGS. 17- 18, the specific structure of the launching key 7 is shown. The launching key 7 is provided with a launching rib 701 extending from the inner wall to the inside, and one end of the launching key 7 is provided with a snap lock 702.

According to an exemplary embodiment of the present disclosure, as shown in FIGS. 19- 20, the specific structure of the tail cap inner core 8 is shown.

The outer wall of the tail cap inner core 8 is provided with a tail cap inner core locating groove 801 extending from the proximal end to the distal end, and the tail cap inner core locating groove 801 has a locating groove end face 802; the outer wall of the distal end of the tail cap inner core 8 is provided with a spiral groove 804, and the near end face of the tail cap inner core 8 is provided with a tail cap inner core hole 806; a plurality of hollow structures 807 are provided around the tail cap inner core hole 806, and the shapes of the hollow structures 807 can be the same or different. In addition, an elastic member 803 is provided on the side wall of the tail cap inner core 8 in a protruding manner, and strip holes extending in the circumferential direction are provided on the front and back sides adjacent to the wall face where the elastic member 803 is located, so that the elastic member 803 has elasticity.

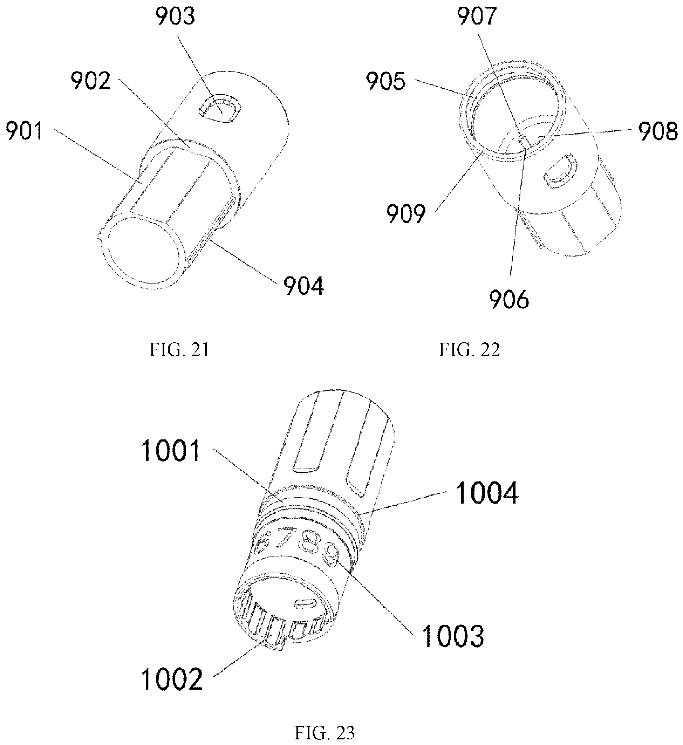

According to an exemplary embodiment of the present disclosure, as shown in FIGS. 21- 22, the specific structure of the tail cap 9 is shown.

The tail cap 9 has a small-diameter part at the proximal end and a large-diameter part at the distal end. A tail cap locating rib 904 is provided on the outer wall face of the small-diameter part, and a tail cap stepped face 902 is provided between the small-diameter part and the large-diameter part; a tail cap rib 906 is also provided on the inner wall face of the small-diameter part, and the distal end of the tail cap rib 906 has a tail cap rib end face 907; the large-diameter part is provided with a viewport 903.

According to an exemplary embodiment of the present disclosure, as shown in FIGS. 23- 25, the specific structure of the shift adjustment key 10 is shown.

The shift adjustment key 10 has adjustment key ribs 1001 provided in the circumferential direction, and its proximal end has adjustment key grooves 1002 provided at intervals in the circumferential direction, and the outer wall face of the proximal end is displayed with an indicator number 1003. The shift adjustment key 10 has a small-diameter part at the proximal end and a large-diameter part at the distal end, and a shift adjustment key stepped face 1004 is provided between the small-diameter part and the large-diameter part. The inner wall of the shift adjustment key 10 is provided with boss structures 1005 protruding radially inward, and the number of the boss structures 1005 can be one, two or more.

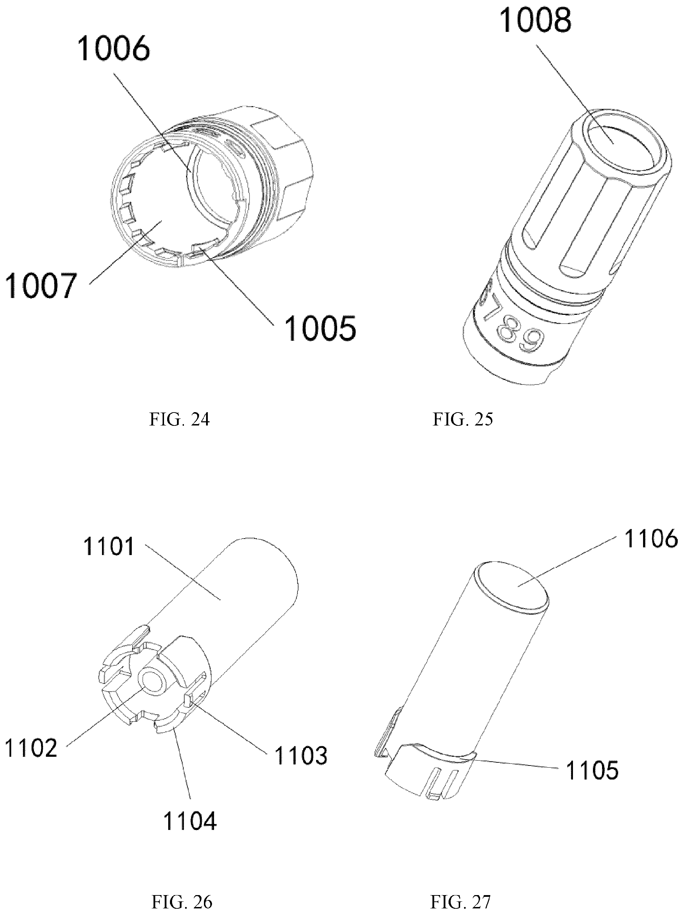

According to an exemplary embodiment of the present disclosure, as shown in FIGS. 26- 27, the specific structure of the needle withdrawing key 11 is shown.

The proximal end of the needle withdrawing key 11 is provided with needle withdrawing key elastic arms 1103 provided at intervals along the axial direction, the middle of the proximal end is provided with a front end central structure 1102, and the outer wall face of the needle withdrawing key 11 is provided with a needle withdrawing key step 1105.

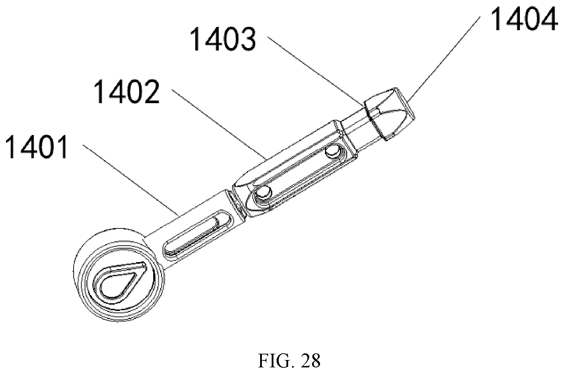

According to an exemplary embodiment of the present disclosure, as shown in FIG. 28, the specific structure of the blood collection needle 14 is shown. The blood collection needle 14 includes a needle cap 1401 and a needle body 1402.

Next, the assembly of the blood collection pen of the present disclosure will be described with reference to the attached drawings:

The inner core 2 has an inner core cavity 214, and the needle retraction rod 3 is assembled inside the inner core 2 and partially wrapped in the inner core cavity 214. The first blocking part 215 of the inner core 2 is matched with the blocking matching portion of the needle retraction rod 3 to prevent the needle retraction rod 3 from coming out of the inner core cavity 214.

Optionally, the number of the first blocking parts 215 may be one or more.

A needle -retraction -rod locating rib 303 is provided on the outer wall of the needle retraction rod 3 to prevent the needle retraction rod 3 from rotating relative to the inner core 2. The needle retraction rod top end 301 passes through the inner core hole 212. Relative to the inner core 2, the needle retraction rod 3 can move forward until the needle retraction rod rear end 306 contacts the inner core tail end face 217, and the needle retraction rod 3 can move backward until the second blocking matching portion 305 of the needle retraction rod contacts the first blocking part 215 of the inner core.

The guide sleeve 5 is assembled in the cavity of the main body 6, and the guide sleeve locating groove 512 is matched with the main body locating rib 603, so that the guide sleeve 5 and the main body 6 cannot rotate relatively.

The locating face 505 of the guide sleeve and the locating member 504 are matched with the circumferential rib 602 of the main body 6, so that the guide sleeve 5 cannot move axially relative to the main body 6.

The front end of the guide sleeve 5 is equipped with a pen cap 1, and the inner wall of the pen cap 1 is provided with a pen cap protrusion 101, which is matched with the clamping groove 503 of the guide sleeve 5, so that the position of the pen cap 1 and the guide sleeve 5 is relatively fixed. When the pen cap 1 is screwed off counterclockwise, the pen cap protrusion 101 can be disengaged from the clamping groove 503 of the guide sleeve 5, so that the pen cap 1 disengages from the guide sleeve 5.

The inn core 2 is assembled in that inn cavity of the guide sleeve 5, and the locating rib 203 of the inner core 2 is matched with the groove 508 of the guide sleeve, so that the inn core 2 can move axially in the cavity of the guide sleeve 5, but cannot rotate circumferentially.

A first spring 12 is assembled on the inner core distal end 210, and one end of the first spring 12 is in contact with the inner core abutting part 216, and the other end is in contact with the guide sleeve abutting face 511. After assembling the first spring 12, the inner core distal end 210 passes through the guide sleeve hole 507 and the tail cap inner core hole 806. Then, a second spring 13 is installed at the inner core distal end 210, a blocking member 4 is installed at the tail of the inner core 2, and a blocking member hole 401 is clamped in the inner core groove 209, so that the blocking member 4 and the inner core 2 are relatively fixed; one end of the second spring 13 is in contact with the tail cap inner core abutting end face 805, and the other end is in contact with a blocking member abutting face 404. The blocking member 4 has a blocking member groove 402, so that when the blocking member 4 is assembled, the blocking member hole 401 can be deformed outward to facilitate the assembly.

The main body 6 is equipped with a launching key 7 for releasing the loaded state of the inner core 2. The snap lock 702 of the launching key 7 engages with the main body key hole 606 to prevent the launching key 7 from coming loose from the main body 6.

The tail cap front end 901 is assembled in a rear cavity of the main body 6, and the main body locating groove 604 is matched with the tail cap locating rib 904 to prevent the tail cap 9 from rotating relative to the main body 6, so that the tail cap 9 and the main body 6 can move relative to each other in the axial direction. In the assembled state, the tail cap stepped face 902 is in contact with the main body rear end face 605.

A tail cap inner core 8 is assembled in the tail cap cavity 908, and the tail cap inner core locating groove 801 is matched with the tail cap rib 906, so that the tail cap inner core 8 and the tail cap 9 can move relatively in the axial direction, but cannot rotate relatively in the circumferential direction. In the assembled state, the tail cap rib end face 907 of the tail cap rib 906 contacts the locating groove end face 802 of the tail cap inner core locating groove 801. There is a hollow structure 807 at the bottom of the tail cap inner core 8. During the use of the blood collection pen, the blocking member 4 will hit the tail cap inner core abutting end face 805, and this hollow structure 807 can effectively reduce the vibration.

The rear part of the cavity of the tail cap 9 is also equipped with a shift adjustment key 10. The shift adjustment key rib 1001 is matched with the tail cap inner wall groove 905 so that the shift adjustment key 10 and the tail cap 9 can rotate relatively in the circumferential direction, but cannot move relatively in the axial direction. In the assembled state, the shift adjustment key stepped face 1004 is in contact with the tail cap rear end face 909. The inner wall of the shift adjustment key 10 is provided with a boss structure 1005, which is matched with the spiral groove 804 of the tail cap inner core; when the shift adjustment key 10 is rotated, the shift adjustment key 10 rotates relative to the tail cap inner core 8. Under the matching of the boss structure of the shift adjustment key 1005 and the spiral groove 804 of the tail cap inner core, because the tail cap inner core locating groove 801 is matched with the tail cap rib 906, the tail cap inner core 8 and the tail cap 9 can only move relatively in the axial direction, so the shift adjustment key 10 can drive the tail cap inner core 8 to move relatively in the axial direction relative to the shift adjustment key 10, thereby limiting the forward movement distance of the inner core 2 and controlling the length of the needle tip of the blood collection needle exposed from the pen cap hole 102.

The shift adjustment key 10 is rotated to rotate the tail cap inner core 8 to the nearest end relative to the tail cap 9 and the shift adjustment key 10. When the blood collection needle 14 moves to the frontmost end relative to the pen cap 1, the needle tip of the blood collection needle 14 is exposed from the end face of the pen cap 1 for the longest distance and penetrates the skin for the deepest depth; the shift adjustment key 10 is rotated to rotate the tail cap inner core 8 to the farthest end relative to the tail cap 9 and the shift adjustment key 10, and when the blood collection needle 14 moves to the frontmost end relative to the pen cap 1, the needle tip of the blood collection needle 14 is exposed from the end face 104 of the pen cap for the shortest distance and penetrates the skin for the shallowest depth.

The blood collection pen related to this disclosure adopts the shift adjustment key 10 on the tail cap 9 to adjust the exposed length of the blood collection needle, which is very convenient.

Adjustment key grooves 1002 are distributed on the inner wall of the shift adjustment key 10, which are matched with the elastic member 803 of the tail cap inner core during the rotation process to generate a shifting sense. There is a shift indicator number 1003 on the outer wall of the shift adjustment key 10. During the rotation of the shift adjustment key 10, the number display can be seen in the tail cap viewport 903.

A needle withdrawing key 11 is assembled in the cavity of the shift adjustment key 10. The needle withdrawing key rear end 1101 passes through the shift adjustment key hole 1008, and the needle withdrawing key 11 can move in the cavity of the shift adjustment key 10. In the assembled state, it can move forward until the needle withdrawing key front end face 1104 contacts with the tail cap inner core rear end face 808, and move backward until the needle withdrawing key step 1105 contacts with the rear end step face 1006 of the shift adjustment key. The needle withdrawing key elastic arm 1103 slightly rubs against the shift adjustment key inner wall 1007, so that the needle withdrawing key 11 and the shift adjustment key 10 can be in a relatively fixed state.

Next, the use process of the blood collection pen of the present disclosure will be explained with the attached drawings.

When the blood collection pen is not equipped with needle and loaded:

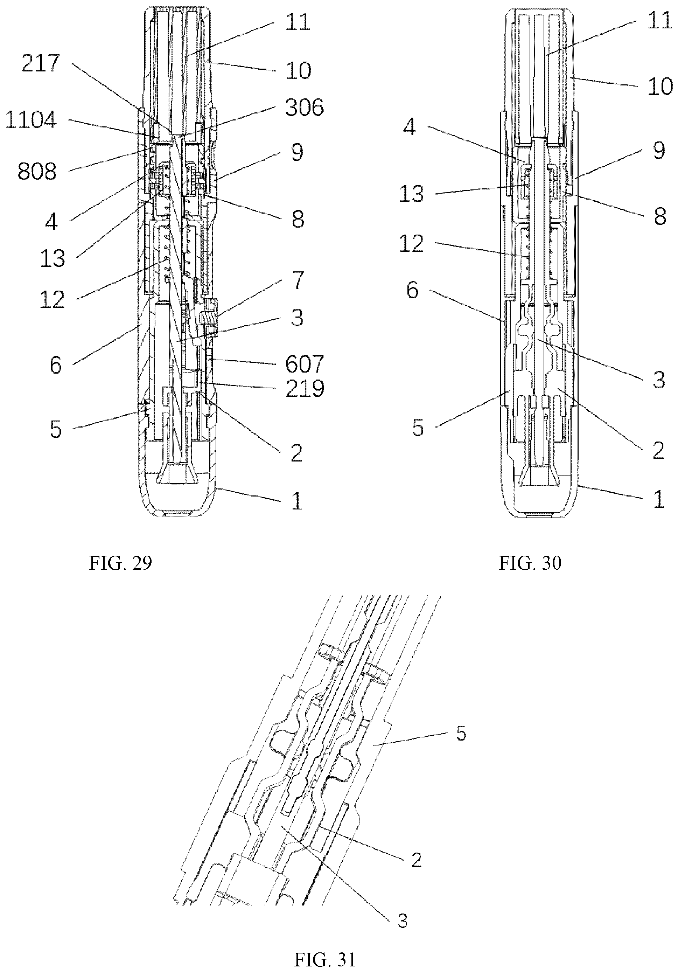

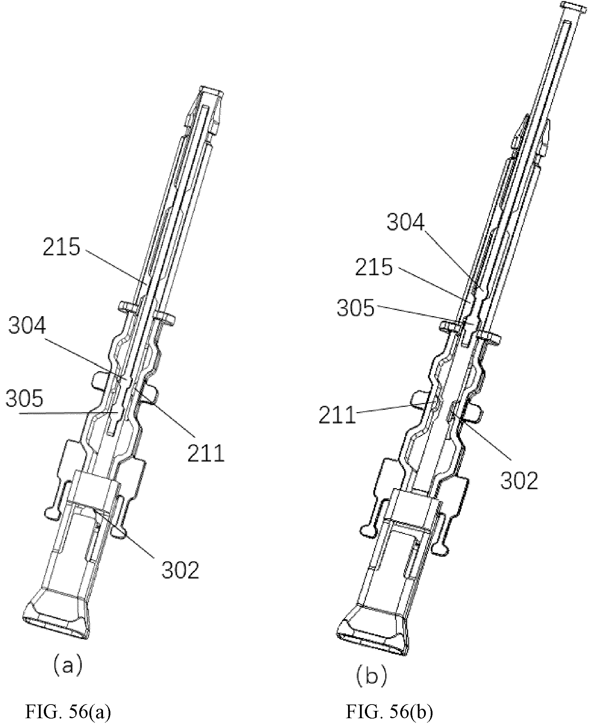

As shown in FIG. 29 and FIG. 30, under the force of the first spring 12 and the second spring 13, the positions and structures of the inner core 2, the first spring 12, the second spring 13, the blocking member 4, the needle retraction rod 3 and the guide sleeve 5 is as shown in the figures. Among them, the inner core 2, the needle retraction rod 3 and the guide sleeve 5 are in the positions shown in FIG. 31, and the positional relationship between the inner core 2 and the needle retraction rod 3 is shown in FIG. 56(a).

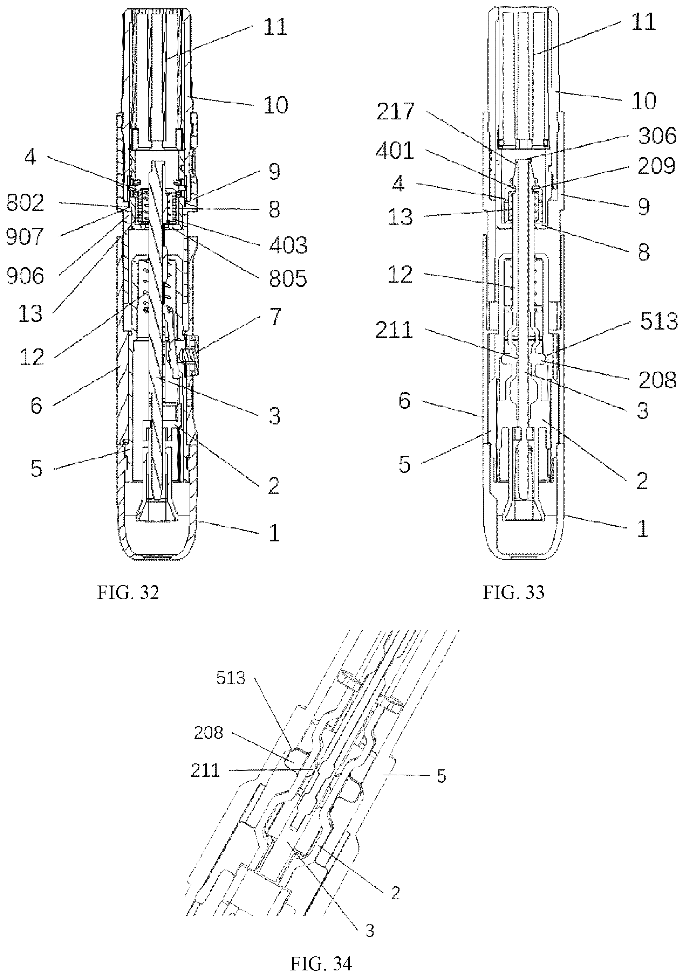

As shown in FIG. 29, the needle retraction rod rear end 306 is in contact with the rear end face 217 of the inner core, and the front end face 1104 of the needle withdrawing key 11 is in contact with the tail cap inner core rear end face 808. At this time, if the tail cap 9 is pulled backwards, the end face 907 of the tail cap rib 906 pushes the locating groove end face 802 of the tail cap inner core locating groove 801, and drives the tail cap inner core 8 to move towards the rear end. Then, the second spring 13 starts to compress under the action of the tail cap inner core 8 until the tail cap inner core abutting end face 805 contacts the front end face 403 of the blocking member, which drives the blocking member 4 to move towards the rear end. Because the blocking member hole 401 is clamped in the inner core groove 209, the blocking member 4 and the inner core 2 remain relatively fixed, so the inner core 2 also moves to the rear end together. The rear end face 217 of the inner core 2 pushes the needle retraction rod rear end 306, so that the needle retraction rod 3 moves to the rear end together, and the positions of the needle retraction rod 3 and the inner core 2 are relatively fixed, as shown in FIG. 32.

When the inner core 2 moves until the first axial limit matching portion 208 contacts with the first axial limit part 513, the first axial limit matching portion 208 interacts with the first axial limit part 513, so that the inner core elastic structure 207 begins to deform inward.

Optionally, at least one contact surface between the first axial limit matching portion 208 and the first axial limit part 513 is inclined, so that under the action of axial force, the two structures will interact with each other and generate lateral displacement, thus making the inner core elastic structure 207 deform inward.

In an alternative embodiment, the contact surface between the first axial limit matching portion 208 of the inner core 2 and the first axial limit part 513 is inclined. Therefore, when the inner core 2 is deformed inward until the lateral limit part 211 contacts with the needle retraction rod 3, the elastic structure 207 of the inner core can no longer be deformed inward, and at this time, the first axial limit matching portion 208 of the inner core is in an interference state with the first axial limit part 513, and the inner core 2 can no longer move to the rear end; the blood collection pen cannot be operated to be loaded and ready to be launched, as shown in FIG. 33 and FIG. 34.

The blood collection pen of the present disclosure includes a safety protection mechanism, which includes an inner core 2, a needle withdrawal bar 3 and a guide sleeve 5. According to the blood collection pen of the embodiment of the present disclosure, when a blood collection needle is not installed on the blood collection pen or the blood collection needle is not installed in place, the blood collection pen cannot be loaded and launched, thus avoiding the blood collection needle from popping out of the pen cap hole to cause harm to the user.

When the blood collection pen is equipped with a blood collection needle:

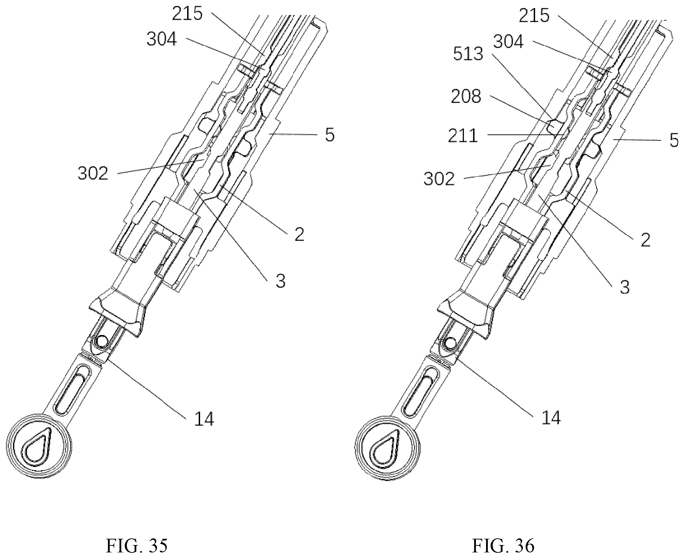

As shown in FIGS. 35 and 36, the blood collection needle 14 is installed through the pen cap hole 102, and the needle 14 pushes the needle retraction rod 3 to move towards the rear end of the inner core 2. In the drawing, the structure of the pen cap 1 is not shown for the convenience of viewing the internal structure.

In the blood collection pen described in this disclosure, the blood collection needle 14 is directly installed into the mounting base 201 from the pen cap 1, so that the use steps are greatly reduced without disassembling the pen cap 1, and the operation convenience of the blood collection pen is significantly improved. Moreover, in the use process of the blood collection pen, except that the needle tip will be exposed from the pen cap hole 102 at the moment of blood collection, the needle tip in all other operation steps will be protected from accidental injury to the user, thus greatly improving the use safety.

According to an embodiment of the present disclosure, when the first blocking matching portion 304 of the needle retraction rod 3 contacts with the first blocking part 215, the needle retraction rod 3 pushes the inner core 2 to move toward the rear end together, as shown in FIG. 35. When the inner core 2 moves such that the first axial limit matching portion 208 comes into contact with the first axial limit part 513, as mentioned above, because the contact surfaces of the first axial limit matching portion 208 of the inner core 2 and the first axial limit part 513 are all inclined planes, the elastic structure 207 of the inner core begins to deform inward under the axial external force. When the inner core is deformed such that the lateral limit part 211 contacts with the needle retraction rod 3, the elastic structure 207 of the inner core can no longer be deformed inward. At this time, the first axial limit matching portion 208 of the inner core is in an interference state with the first axial limit part 513, and the inner core 2 can no longer move to the rear end, as shown in FIG. 36.

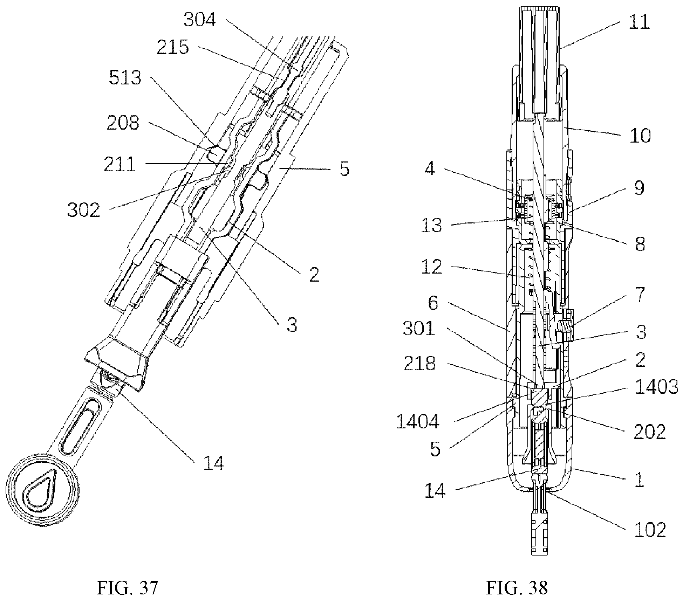

The needle retraction rod 3 is continually to be pushed to the rear end, so that the first blocking matching portion 304 of the needle retraction rod and the first blocking part 215 of the inner core interact with each other. The first blocking matching portion 304 exerts a force on the first blocking part 215, and under the action of axial force, the two structures interact with each other and generate lateral displacement, so that the first blocking part 215 begins to change outward, and the first blocking matching portion 304 of the needle retraction rod passes through the first blocking part 215 of the inner core, as shown in FIG. 37 and FIG. 56(b).

Optionally, at least one contact surface between the first blocking matching portion 304 and the first blocking part 215 of the inner core is an inclined plane.

In an alternative embodiment, the contact surfaces of the first blocking matching portion 304 and the first blocking part 215 of the inner core are all inclined planes to facilitate the interaction between the first blocking matching portion 304 and the first blocking part 215 of the inner core, so that the first blocking matching portion 304 can easily cross the blocking of the first blocking part 215 of the inner core.

The first blocking matching portion 304 of the needle retraction rod passes through the first blocking part 215 of the inner core until the end face of the needle retraction rod top end 301 is flush with the inner core bottom face 218. At this time, the bottom face 1404 of the blood collection needle contacts with the inner core bottom face 218, and the blood collection needle stepped face 1403 is clamped with the elastic arm structure 202 of the inner core, so that the positions of the blood collection needle 14 and the inner core 2 are relatively fixed in a clamping state, as shown in FIG. 38. In this state, the needle retraction rod avoidance space 302 is at the same horizontal position as the inner core lateral limit part 211, so that the inner core lateral limit part 211 obtained a space for moving inward, as shown in FIG. 37 and FIG. 56(b).

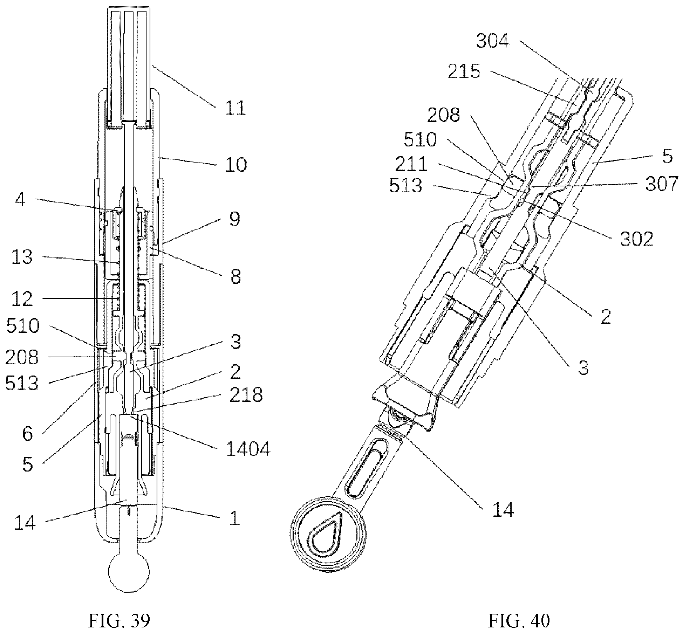

The blood collection needle 14 is continually to bed pushed to the rear end, and the blood collection needle bottom face 1404 pushes the inner core bottom face 218, so that the inner core 2 continues to move to the rear end. During the movement of the inner core 2, the elastic structure 207 of the inner core continues to deform inward until the first axial limit matching portion 208 of the inner core 2 slides over the first axial limit part 513 and contacts the inner wall 510 of the guide sleeve, as shown in FIGS. 39 and 40.

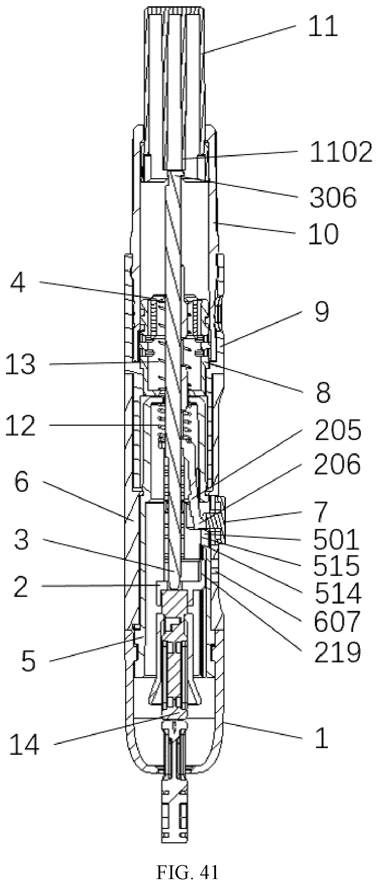

The avoidance depth of the needle retraction rod avoidance space 302 in the transverse direction is greater than the interference between the first axial limit matching portion 208 of the inner core and the first axial limit part 513 in the lateral position. During this movement, the elastic arm end part 206 of the inner core elastic arm 205 comes into contact with the protruding inclined plane 514 of the guide sleeve 5, and under its action, the elastic arm 205 deforms in the axial direction of the blood collection pen and slides over the inner wall of the first convex 515 of the guide sleeve until it slides over the guide sleeve stepped face 501, and then the inner core elastic arm 205 returns to the natural state outward. At this time, in the transverse direction, the elastic arm end part 206 is in an interference state with the guide sleeve stepped face 501, as shown in FIG. 41.

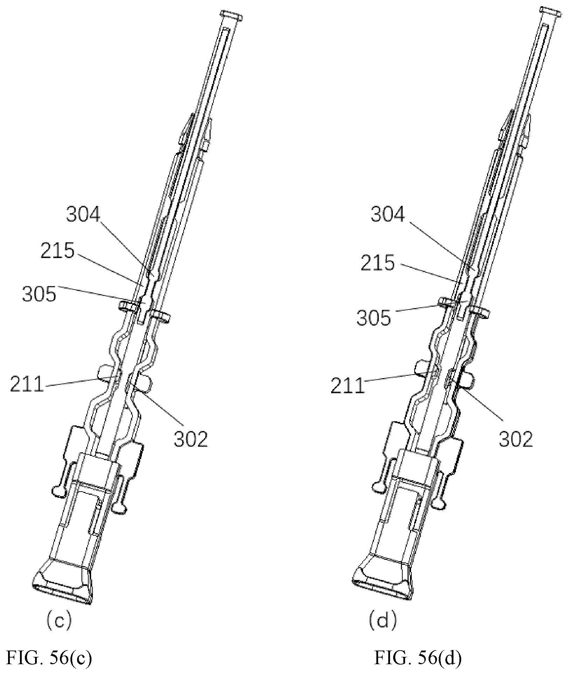

When the inner core 2 moves until the rear end face of the inner core locating rib 203 contacts with the groove bottom end face 509 of the guide sleeve, the inner core 2 cannot continue to move backward. The external force applied to the blood collection needle 14 is cancelled, and at this time, because the first spring 12 is in a compressed state, the inner core 2 starts to move to the front end under the thrust of the first spring 12 until the elastic arm end part 206 contacts the guide sleeve stepped face 501. At this time, the first spring 12 is compressed and acts on the inner core abutting part 216, and the blood collection pen is loaded and ready for launching, as shown in FIG. 41. At this time, the positional relationship between the inner core 2 and the needle retraction rod 3 is as shown in FIG. 56(c). The needle retraction rod avoidance space 302 and the inner core structure 211 are in the same horizontal position, and the first blocking part 215 is located between the first blocking matching portion 304 and the second blocking matching portion 305.

When the blood collection pen is in the loaded state, the inner core protrusion 219 is at the same level as the main body sight hole 607. Alternatively, the guide sleeve 5 is a transparent member, so that the inner core protrusion 219 can be seen through the main body sight hole 607, as shown in FIG. 41. However, in the unloaded state before use, the inner core protrusion 219 is located closer to the front end relative to the main body sight hole 607, and the inner core protrusion 219 cannot be seen through the main body sight hole 607, as shown in FIG. 29. At this time, the inner core hollow part 204 is seen through the main body sight hole 607.

Alternatively, if the inner core protrusion 219 is colored, the color difference can be seen through the main body sight hole 607, so as to judge whether the blood collection pen is loaded or not.

According to the exemplary embodiment of the present disclosure, there is another loading mode for the blood collection pen of the present disclosure.

A blood collection needle 14 is installed through the cap hole 102, and the blood collection needle 14 pushes the needle retraction rod 3 to move towards the rear end of the inner core 2. When the first blocking matching portion 304 of the needle retraction rod 3 comes into contact with the first blocking part 215, the needle retraction rod 3 pushes the inner core 2 to move towards the rear end together, until the first axial limit matching portion 208 comes into contact with the first axial limit part 513, the two parts interfere with each other and the inner core 2 stops moving backwards. The needle retraction rod 3 is continually to be pushed, and the first blocking matching portion 304 of the needle retraction rod interacts with the first blocking part 215 of the inner core, so that the first blocking matching portion 304 passes through the first blocking part 215 of the inner core until the end face of the needle retraction rod tip 301 is flush with the inner core bottom face 218. At this time, the blood collection needle stepped face 1403 is clamped with the inner core elastic arm structure 202, and the needle retraction rod avoidance space 302 is at the same horizontal position as the inner core lateral limit part 211, so that the inner core lateral limit part 211 obtains a space for moving inward.

After the blood collection needle 14 and the inner core 2 are clamped, at this time, the external force applied to the blood collection needle 14 is cancelled, and then the tail cap 9 is pulled to the rear end, the end face 907 of the tail cap rib pushes the locating groove end face 802 of the tail cap inner core locating groove 801, thereby driving the tail cap inner core 8 to move to the rear end. Then, the second spring 13 starts to compress under the action of the tail cap inner core 8 until the tail cap inner core abutting end face 805 contacts the front end face 403 of the blocking member, which drives the blocking member 4 to move towards the rear end. Because the blocking member hole 401 is clamped in the inner core groove 209, the blocking member 4 and the inner core 2 are relatively fixed, therefore the inner core 2 also moves to the rear end together until the elastic arm end part 206 of the inner core 2 contacts the guide sleeve stepped face 501, at which time the blood collection pen is loaded and ready to be launched. The tail cap 9 is loosened, and the tail cap 9 returns to the normal state under the action of the second spring 13, as shown in FIG. 41.

During the whole process from the beginning of installing the blood collection needle 14 until the blood collection needle is loaded, the needle retraction rod rear end 306 keeps in contact with the front end central structure 1102 of the needle withdrawing key, so that the needle withdrawing key 11 moves backward relative to the shift adjustment key 10.

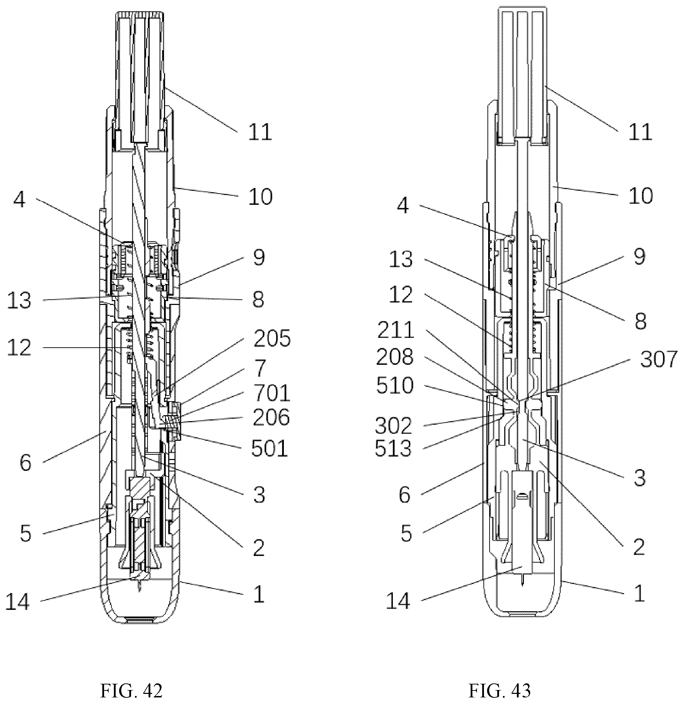

The needle cap 1401 is twisted off, and the blood collection pen is in the state of waiting for launching and blood collection, as shown in FIG. 42 and FIG. 43.

When the blood collection pen is in the state of waiting for launching and waiting for blood collection, if the launching key 7 is not pressed at this time, but only the needle withdrawing key 11 is pushed forward, the front end central structure 1102 of the needle withdrawing key 11 pushes the needle withdrawal bar rear end 306, so that the needle withdrawal bar 3 moves forward. When the concave side wall 307 contacts with the inner core lateral limit part 211, the inner core lateral limit part 211 pushes the inner core elastic structure 207 to begin to deform to the outer shape under the force of the concave side wall 307.

Optionally, at least one contact surface between the concave side wall 307 and the inner core lateral limit part 211 is inclined, so that under the action of axial force, the two structures will interact and generate lateral displacement, thus making the inner core elastic structure 207 deform outward.

In an alternative embodiment, the contact surfaces of the concave side wall 307 and the inner core lateral limit part 211 are all inclined planes.

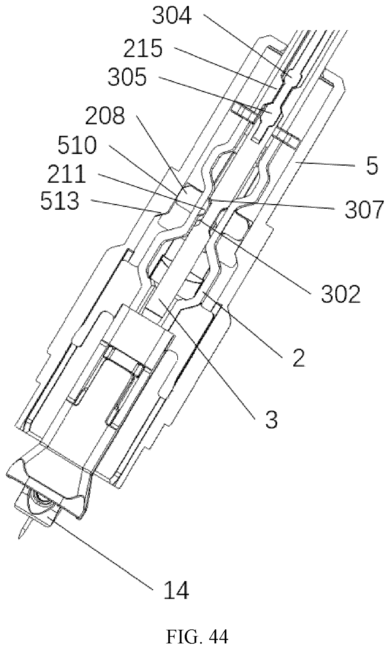

Under the force of the concave side wall 307, the inner core lateral limit part 211 pushes the inner core elastic structure 207 to begin to deform to the outer shape. In this state, the first axial limit matching portion 208 on the inner core elastic structure 207 is in contact with the inner wall 510 of the guide sleeve, therefore the inner core elastic structure 207 has no room for outward deformation, and the concave side wall 307 is in an interference state with the inner core lateral limit part 211, so that the needle withdrawal bar 3 cannot move forward any more, and at the same time, the needle withdrawing key 11 cannot move forward under the action of external force, as shown in FIG. 40, FIG. 43 and FIG. 44.

The needle withdrawing mechanism of the blood collection pen disclosed by the invention has the function of preventing misoperation, which can avoid the situation that the needle withdrawing key 11 is pressed due to the misoperation of the user, so that the blood collection needle 14 is pushed out of the pen cap hole 102, and the needle tip of the blood collection needle causes accidental injury to the user when the blood collection pen is in the state of waiting for blood collection, that is, when the blood collection pen is in the loaded state; it can also avoid the situation that when the blood collection pen is in the to-be-launched state (the needle cap 1401 of the blood collection needle is not unscrewed), the blood collection needle 14 is pushed out of the pen cap hole 102 due to the misoperation of the user by pressing the needle withdrawing key 11, which may otherwise cause the user to reinstall the blood collection needle and lead to repeated actions. That is, in the blood collection pen of the present disclosure, when the launching key 7 is not pressed, the needle withdrawing key cannot be pressed down to withdraw the needle.

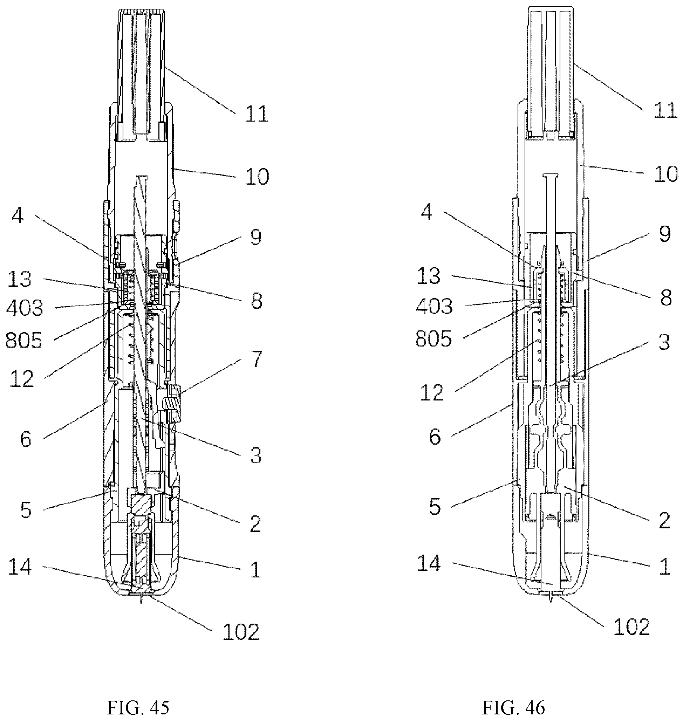

When the launching key 7 is pressed, the end face of the launching rib 701 pushes the inner core elastic arm end part 206 inward, so that the inner core elastic arm 205 bends inward and deforms, and the elastic arm end part 206 is no longer engaged with the guide sleeve stepped face 501, and the inner core 2 moves forward axially under the action of the first spring 12 until the first spring 12 is completely released. Then, under the inertia, the inner core 2 continues to move axially forward until the front end face 403 of the blocking member contacts the tail cap inner core abutting end face 805, and the inner core 2 stops moving forward. At this time, the needle tip of the blood collection needle 14 has been exposed from the pen cap hole 102, and the blood collection operation is completed, as shown in FIGS. 45, 46 and 47; when the needle tip of the blood collection needle is pricked out, the positional relationship between the inner core 2 and the needle withdrawing key 3 is as shown in FIG. 56(d), the needle retraction rod avoidance space 302 and the inner core structure 211 are in the same horizontal position, and the first blocking part 215 is located between the first blocking matching portion 304 and the second blocking matching portion 305.

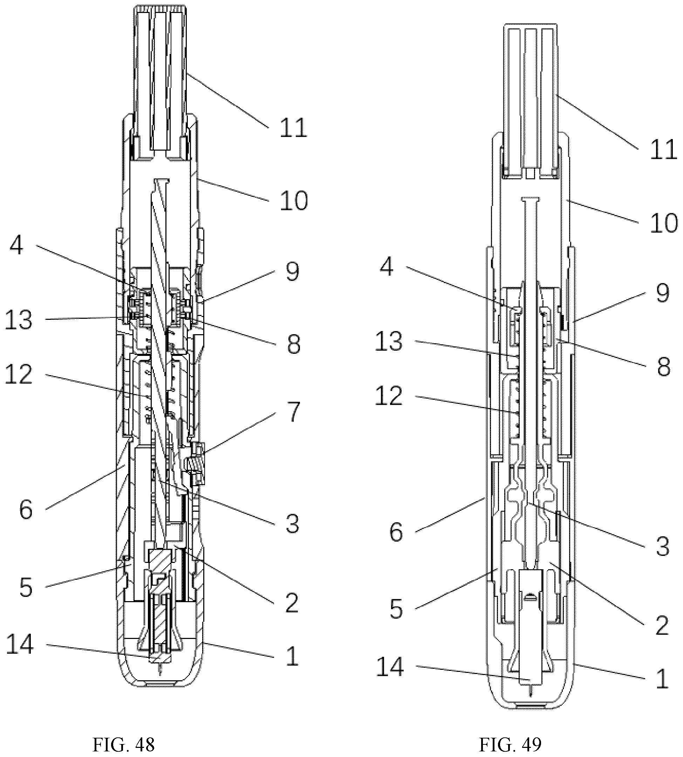

Then, under the action of the second spring 13, the blocking member 4 drives the inner core 2 to move backward until it moves to an unloaded state, as shown in FIGS. 48 and 49.

In the whole process of the forward and backward movement of the inner core 2, the end part of the elastic arm structure 202 is clamped with the blood collection needle stepped face 1403, so that the blood collection needle 14 and the inner core 2 are always in a relatively fixed state.

In the whole process of the forward movement of the inner core 2, the first blocking part 215 of the inner core 2 pushes the second blocking matching portion 305 of the needle retraction rod, so that the needle retraction rod 3 moves forward with the inner core 2. When the blocking member 4 contacts the tail cap inner core abutting end face 805, and the inner core 2 stops moving forward, the first stop matching portion 304 of the needle retraction rod is in contact with the first blocking part 215, so that the needle retraction rod 3 will not continue to move forward relative to the inner core 2 due to inertia, thus pushing the blood collection needle 14 forward, so that the blood collection needle stepped face 1403 14 and the inner core elastic arm structure 202 will not be loosened, and the blood collection needle 14 will not be loosened.

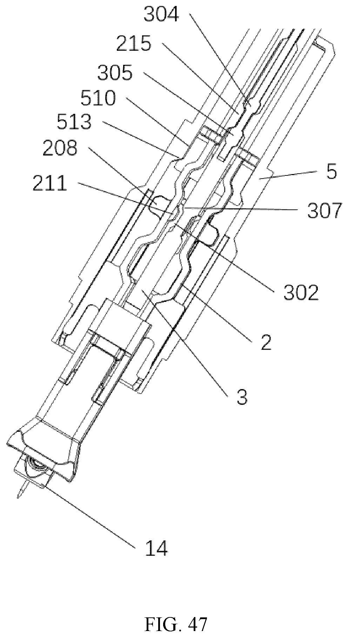

During the backward movement of the inner core 2, the first blocking part 215 of the inner core 2 pushes the first blocking matching portion 304, so that the needle retraction rod 3 moves backward together with the inner core 2. After the backward movement of the inner core 2 stops, the second stop matching portion 305 of the needle retraction rod contacts with the first blocking part 215 of the inner core, so that the needle retraction rod 3 will not continue to move backward relative to the inner core 2 due to inertia, thus avoiding the relative position of the needle retraction rod 3 and the inner core 2 from changing, as shown in FIG. 47. The needle withdrawing key 11 does not participate in the movement of the inner core 2.

After blood collection is completed, the needle is ready to be withdrawn.

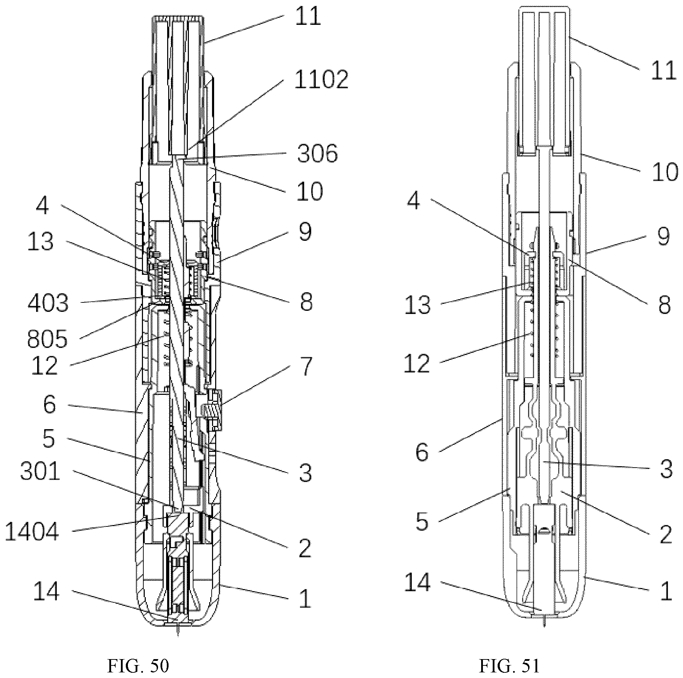

At this time, the needle withdrawing key 11 is pushed, so that the needle withdrawing key 11 moves axially forward relative to the shift adjustment key 10. When the front end central structure 1102 of the needle withdrawing key contacts the needle withdrawal bar rear end 306, the needle withdrawing key 11 pushes the needle withdrawal bar 3 to move forward together. Because the first blocking matching portion 304 of the needle retraction rod is in contact with the first blocking part 215 of the inner core (see FIG. 47, for example), the needle retraction rod 3 drives the inner core 2 to move forward together, and at the same time, the blocking member 4 and the blood collection needle 14 also move forward together with the inner core 2. When the front end face 403 of the blocking member contacts the tail cap inner core abutting end face 805 805, the inner core 2 cannot move forward any more because the front end of the tail cap inner core 8 abuts the rear end of the guide sleeve 5, as shown in FIGS. 50 and 51.

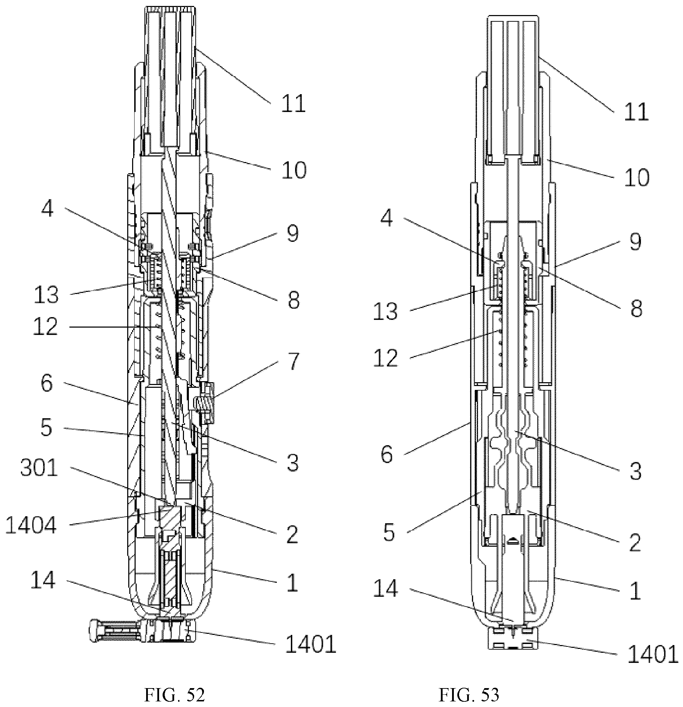

At this time, the needle tip of the blood collection needle 14 is exposed from the cap hole 102. In this state, the needle tip of the blood collection needle can be aligned with the needle cap 1401, so that the needle tip of the blood collection needle can be pierced into the needle cap 1401, as shown in FIGS. 52 and 53.

The needle withdrawing key 11 is continually to be pushed, so that the needle withdrawing key 11 will continue to exert the force on the needle retraction rod 3 to move forward. Because the contact surface between the first blocking matching portion 304 of the needle retraction rod and the first blocking part 215 of the inner core is inclined, the first blocking part 215 of the inner core begins to deform outward under the action of axial force, so that the first blocking matching portion 304 of the needle retraction rod passes through the first blocking part 215 of the inner core, and the needle retraction rod 3 and the inner core 2 are relatively displaced.

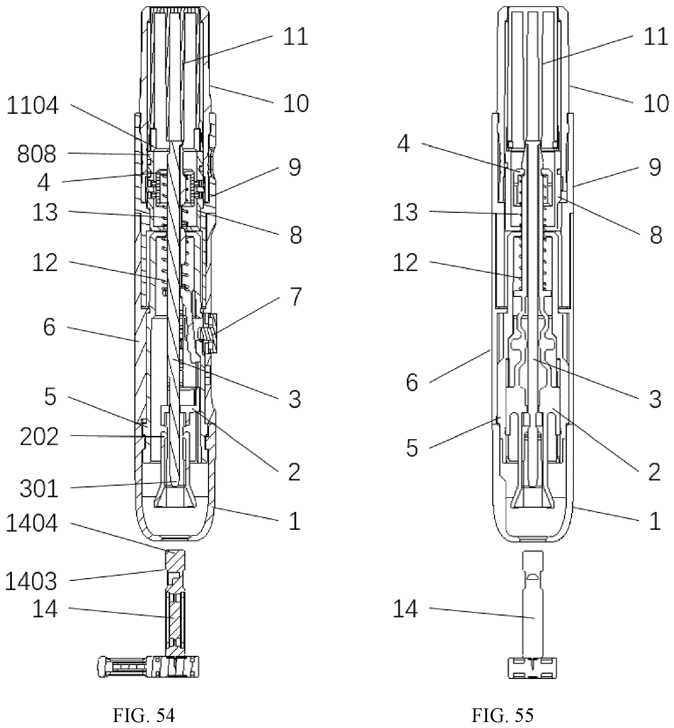

In this process, the needle retraction rod top end 301 pushes the bottom face 1404 of the blood collection needle, so that the blood collection needle stepped face 1403 is released from the inner core elastic arm structure 202, and the blood collection needle 14 is withdrawn from the pen cap hole 102 and freely falls off. After the first blocking matching portion 304 of the needle withdrawal bar is released from the inner core elastic arm structure 202 through the first blocking part 215 of the inner core and the blood collection needle stepped face 1403, the needle withdrawing key 11 is continuously pressed, and the needle withdrawal bar 3 moves forward together under the action of the needle withdrawing key 11 until the needle withdrawing key front end face 1104 contacts the tail cap inner core rear end face 808. Because the inner core 2 is relatively fixed with the blocking member 4, the blocking member 4 drives the inner core 2 to move backward under the action of the second spring 13 until it moves to an unloaded state, as shown in FIGS. 54 and 55.

In the above embodiment, the lateral limit part 211 is provided on the inner side of the elastic structure, but in an alternative embodiment, although not shown, the lateral limit part extending axially is provided on the outer side of the needle retraction rod in the transverse direction, and the lateral limit part is adapted to abut against the elastic structure laterally outward to prevent the first axial limit matching portion from moving laterally inward when the blood collection needle is not installed or not properly installed at the front end of the inner core, and the first axial limit matching portion 208 is adapted to abut against the first axial limit part 513 in the axial direction to prevent the inner core 2 from moving distally to the to-be-launched state. In a case that the outer side of the needle retraction rod in the transverse direction is provided with an axially extending lateral limit part, the lateral inner side of the elastic structure can be provided with an avoidance space; during the installation of the blood collection needle, the blood collection needle 14 is adapted for pushing the needle retraction rod 3 to move towards the distal end in the inner core 2 until the avoidance space is opposite to the lateral limit part in the transverse direction to allow the part of the elastic structure that abuts against the first axial limit part to elastically deform or move inward in the transverse direction, so that the first axial limit matching portion 208 disengages from the abutment against the first axial limit part 513 in the axial direction.

In addition, in this disclosure, the inclined plane structure is provided to form movement interference in the axial direction, and other structures, such as convex surface, can be used besides the inclined plane structure.

Based on the above, the present disclosure proposes the following technical solution:
1. A blood collection pen, comprising:
   a guide sleeve;
   an inner core, a front end of which is provided with a needle mounting mechanism for detachably installing a blood collection needle, and a rear end of which passes through the guide sleeve;
   a needle retraction rod provided through the inner core in an axial direction, a front end of the needle retraction rod being adapted for passing through an inner core hole at a front end of the inner core,
   wherein
   the inn core includes an elastic structure which is adapted for abut against an inner wall of the guide sleeve in the axial direction; and
   the elastic structure is adapted to abut against the needle retraction rod in a transverse direction to prevent a part of the elastic structure that abuts against the inner wall of the guide sleeve from elastically deforming or moving laterally inward in a case that the blood collection needle is not installed or not properly installed at the front end of the inner core.
2. The blood collection pen according to 1, wherein
   a first axial limit matching portion is provided outside the elastic structure in the transverse direction, and the first axial limit matching portion is adapted to abut against a first axial limit part on the inner wall of the guide sleeve in the axial direction;
   the elastic structure is adapted to abut against the needle retraction rod in the transverse direction in a case that the blood collection needle is not installed or not properly installed at the front end of the inner core, so as to prevent the first axial limit matching portion from elastically deforming or moving laterally inward, thereby preventing the first axial limit matching portion from disengaging from abutment against the first axial limit part in the axial direction.
3. The blood collection pen according to 2, wherein
   an axially extending lateral limit part is provided outside the needle retraction rod in the transverse direction, and the lateral limit part is adapted to abut against the elastic structure laterally outward to prevent the first axial limit matching portion from moving laterally inward in a case that the blood collection needle is not installed or not properly installed at the front end of the inner core; and the first axial limit matching portion is adapted to abut against the first axial limit part protruding laterally inward in the axial direction to prevent the inner core from moving toward a distal end to a to-be-launched state.
4. The blood collection pen according to 3, wherein
   an avoidance space is provided inside the elastic structure in the transverse direction;
   in a process of installing the blood collection needle, the blood collection needle is adapted for pushing the needle retraction rod to move towards the distal end in the inner core until the avoidance space is opposite to the lateral limit part in the transverse direction to allow the part of the elastic structure that abuts against the first axial limit part to elastically deform or move inward in the transverse direction, so that the first axial limit matching portion disengages from the abutment against the first axial limit part in the axial direction.
5. The blood collection pen according to 2, wherein the elastic structure is provided with the first axial limit matching portion and the lateral limit part which are respectively located at two sides of the elastic structure in the transverse direction, and the first axial limit matching portion is adapted to abut against the first axial limit part protruding laterally inward in the axial direction; and
   in a case that the blood collection needle is not installed or not properly installed at the front end of the inner core, the lateral limit part is adapted to abut against the needle retraction rod laterally inward to prevent the first axial limit matching portion from moving laterally inward, and the first axial limit matching portion is adapted for axially abutting against the first axial limit part to prevent the inner core from moving to the distal end to the to-be-launched state.
6. The blood collection pen according to 5, wherein
   the needle retraction rod is provided with an avoidance space; and
   in a process of installing the blood collection needle, the blood collection needle is adapted for pushing the needle retraction rod to move towards the distal end in the inner core until the avoidance space is opposite to the lateral limit part in the transverse direction to allow the elastic structure to deform laterally inward, so that the first axial limit matching portion disengages from the abutment against the first axial limit part in the axial direction.
7. The blood collection pen according to 6, wherein
   one side of the elastic structure facing the needle retraction rod is provided with a first blocking part, and the needle retraction rod is provided with a first blocking matching portion;
   with the action of an external force, the needle retraction rod is adapted to move in the inner core until the first blocking matching portion abuts against the first blocking part and moves across the first blocking part, in which case, the avoidance space is opposite to the lateral limit part in the transverse direction.
8. The blood collection pen according to 7, wherein
   in a case that the first blocking matching portion moves across the first blocking part, a front end face of the needle retraction rod is flush with a bottom end face of the inner core.
9. The blood collection pen according to 7, wherein
   the needle retraction rod is provided with a second blocking matching portion axially spaced from the first blocking matching portion and closer to the front end;
   in a case that the front end face of the needle retraction rod is flush with the bottom end face of the inner core, the first blocking part is provided between the first blocking matching portion and the second blocking matching portion in the axial direction, the first blocking matching portion and the second blocking matching portion are adapted for providing a moving resistance for an axial movement of the first blocking part, and the first blocking part is adapted for providing a moving resistance for an axial movement of the first blocking matching portion and the second blocking matching portion .
10. The blood collection pen according to 9, wherein
   at least one of the abutting faces of the second blocking matching portion and the first blocking part in the axial direction is an inclined plane or a convex surface.
11. The blood collection pen according to 7, wherein
   the first blocking matching portion and the first blocking part are adapted for forming an interference in an axial movement.
12. The blood collection pen according to claim 11, wherein
   at least one of the abutting faces of the first blocking matching portion and the first blocking part in the axial direction is an inclined plane or a convex surface.
13. The blood collection pen according to 6, wherein
   the lateral limit part is adapted to be located within the avoidance space; and
   the avoidance space is a concave space, and a side wall near the distal end of the concave space is adapted for interfering with the end face near the distal end of the lateral limit part in axial movement.
14. The blood collection pen according to claim 13, wherein:
   at least one of the axial abutting faces of the side wall near a distal end of the concave space and an end face near a distal end of the lateral limit part is an inclined plane or a convex surface.
15. The blood collection pen according to any one of 6-14, wherein
   based on the lateral inward movement of the lateral limit part towards the avoidance space, the first axial limit matching portion is adapted to disengage from the axial abutment against the first axial limit part, so that the inner core is adapted to move towards the distal end under the action of an external force matching portion.
16. The blood collection pen according to 15, wherein
   the first axial limit matching portion is adapted to abut against or contacting with the inner wall of the guide sleeve to keep the interference fit between the lateral limit matching and the avoidance space in the axial direction after the first axial limit matching portion disengages from the axial abutment against the first axial limit part matching portion.
17. The blood collection pen according to 2, wherein
   the first axial limit matching portion and the first axial limit part are adapted for forming an interference in an axial movement.
18. The blood collection pen according to claim 17, wherein
   at least one of the abutting faces of the first axial limit matching portion and the first axial limit part in the axial direction is a inclined plane or a convex surface.
19. The blood collection pen according to 2, wherein
   in a case that the first axial limit matching portion disengages from the abutment against the first axial limit part in the axial direction, the movement of the inner core towards the distal end is adapted for forming a loaded abutment against the guide sleeve.
20. The blood collection pen according to claim 19, wherein
   the inner core is provided with an elastic arm, the guide sleeve is provided with a first protrusion protruding laterally inward, and a distal end of the first protrusion is provided with a stepped face; and
   in a case that the first axial limit matching portion disengages from the abutment against the first axial limit part in the axial direction, the inner core is adapted for moving to the distal end until an elastic arm end part of the elastic arm moves across the first protrusion to abut against the stepped face in the axial direction, thus forming the loaded abutment.
21. The blood collection pen according to 5, wherein
   the first axial limit matching portion and the lateral limit part are oppositely provided at two sides of the elastic structure in the transverse direction.
22. The blood collection pen according to 1, wherein
   the inner core is provided with an axially extending inner core locating rib the inner wall of the guide sleeve is provided with a guide groove, and the guide groove has a groove bottom end face; and
   the inner core locating rib is adapted to slide in the guide groove until the locating rib abuts against the groove bottom end face.
23. The blood collection pen according to any one of 7-12, wherein
   the needle retraction rod is provided with an axially extending needle -retraction - rod locating rib, and the first blocking matching portion is formed by laterally expanding the needle -retraction -rod locating rib.
24. The blood collection pen according to 9, wherein
   the needle retraction rod is provided with an axially extending needle -retraction - rod locating rib, and the second blocking matching portion is formed by laterally expanding the needle -retraction -rod locating rib.
25. The blood collection pen according to 23, wherein
   the positions of the avoidance space and the needle -retraction -rod locating rib in the circumferential direction of the needle retraction rod differ by 90 degrees.
26. The blood collection pen according to 1, wherein
   the needle retraction rod is provided with an axially extending needle -retraction - rod locating rib, and the guide sleeve is provided with a guide groove for guiding the needle - retraction -rod locating rib to move axially.
27. The blood collection pen according to any one of 1-26, further comprising:
   a tail cap;
   a tail cap inner core axially movable in the tail cap, wherein the tail cap inner core and the tail cap are circumferentially limited by a guide structure; and
   a blocking member axially movable in the tail cap inner core,
   wherein
   a distal end of the inner core is provided with an inner core groove, the distal end of the inner core passes through a guide sleeve hole of the guide sleeve and a tail cap inner core hole of the tail cap inner core in the axial direction, and the blocking member is adapted for being clamped in the inner core groove;
   in a case that the first axial limit matching portion disengages from abutment against the first axial limit part in the axial direction, the tail cap is adapted for driving the tail cap inner core under the action of an axial external force, so that the blocking member and the inner core clamped with the blocking member move axially to the distal end, thereby realizing the loading of the blood collection pen.
28. The blood collection pen according to 27, wherein
   the inn core is provided with an inn core locating rib, the inner wall of the guide sleeve is provided with a guide groove, and the guide groove is provided with a groove bottom end face; and
   the inner core locating rib is adapted to slide in the guide groove until the inner core locating rib abuts against the groove bottom end face.

It should be noted that the above technical solutions can be combined in any logical situation, which shall fall within the protection scope of this disclosure. In this specification, relational terms such as "first" and "second" are only used to distinguish one entity or operation from another entity or operation without necessarily requiring or implying any such actual relationship or order between these entities or operations. Moreover, the terms "including", "comprising" or any other variation thereof are intended to cover non-exclusive inclusion, so that a process, method, article or equipment including a series of elements includes not only those elements, but also other elements not explicitly listed or elements inherent to such process, method, article or equipment. Without further restrictions, an element defined by the phrase "including one" does not exclude the existence of other identical elements in the process, method, article or equipment including the element.

Each embodiment in this specification is described in a related way, and only the same and similar parts between the embodiments can be referred to each other, and each embodiment focuses on the differences from other embodiments.

Although embodiments of the present disclosure have been shown and described, it will be appreciated by those skilled in the art that variations and combinations of elements can be made in these embodiments without departing from the principles and spirit of the present disclosure, and the scope of the present disclosure is defined by the appended claims and the equivalents thereof.

## Claims

1. A blood collection pen, comprising:
a guide sleeve (5);
an inner core (2), a front end of which is provided with a needle mounting mechanism for detachably installing a blood collection needle, and a rear end of which passes through the guide sleeve;
a needle retraction rod (3) which is provided to pass through the inner core in an axial direction, a front end of the needle retraction rod being adapted to pass through an inner core hole at the front end of the inner core,
wherein:
the inn core includes an elastic structure (207) which is adapted to abut against an inner wall of the guide sleeve in the axial direction; and
the elastic structure (207) is adapted to abut against the needle retraction rod (3) in a lateral direction to prevent the part of the elastic structure that abuts against the inner wall of the guide sleeve from elastically deforming or moving laterally inward in a case that the blood collection needle is not installed or not properly installed at the front end of the inner core.

2. The blood collection pen according to claim 1, wherein:
a first axial limit matching portion (208) is provided outside the elastic structure (207) in the lateral direction, and the first axial limit matching portion is adapted to abut against a first axial limit portion (513) on the inner wall of the guide sleeve in the axial direction;
the elastic structure (207) is adapted to abut against the needle retraction rod (3) in the transverse direction in a case that the blood collection needle is not installed or not properly installed at the front end of the inner core, so as to prevent the first axial limit matching portion (208) from elastically deforming or moving laterally inward and to prevent the first axial limit matching portion from disengaging from abutment against the first axial limit portion in the axial direction; optionally,
the needle retraction rod (3) is provided with an axially extending needle -retraction -rod locating rib (303), and the guide sleeve is provided with a guide groove for guiding the needle -retraction -rod locating rib to move axially.

3. The blood collection pen according to claim 2, wherein:
an axially extending lateral limit portion is provided outside the needle retraction rod in the lateral direction, and the lateral limit portion is adapted to abut against the elastic structure laterally outward to prevent the first axial limit matching portion from moving laterally inward in a case that the blood collection needle is not installed or not properly installed at the front end of the inner core; and
the first axial limit matching portion is adapt to abut against the first axial limit portion, which protrudes laterally inward, in the axial direction to prevent the inner core from moving toward a distal end to a to-be-launched state; optionally,
an avoidance space is provided inside the elastic structure in the lateral direction;
during the installation of the blood collection needle, the blood collection needle is adapted to push the needle retraction rod to move towards the distal end in the inner core until the avoidance space is opposite to the lateral limit portion in the lateral direction, allowing the part of the elastic structure that abuts against the first axial limit portion to elastically deform or move inward in the lateral direction, so that the first axial limit matching portion disengages from the first axial limit portion in the axial direction.

4. The blood collection pen according to claim 2, wherein:
the elastic structure (207) is provided with the first axial limit matching part (208) and the lateral limit part (211) which are respectively located at two sides of the elastic structure in the lateral direction, and the first axial limit matching part (208) is adapted to abut against the first axial limit part (513) protruding laterally inward in the axial direction; and
in a case that the blood collection needle is not installed or not properly installed at the front end of the inner core, the lateral limit part (211) is adapted to abut against the needle retraction rod laterally inward to prevent the first axial limit matching part (208) from moving laterally inward, and the first axial limit matching part is adapted to axially abut against the first axial limit part (513) to prevent the inner core from moving to the distal end to the to-be-launched state.

5. The blood collection pen according to claim 4, wherein:
the needle retraction rod is provided with an avoidance space (302); and
in a process of installing the blood collection needle, the blood collection needle is adapted to push the needle retraction rod to move towards the distal end in the inner core until the avoidance space (302) is opposite to the lateral limit part (211) in the lateral direction to allow the elastic structure (207) to deform laterally inward, so that the first axial limit matching part (208) is disengaged from the abutment against the first axial limit part (513) in the axial direction.

6. The blood collection pen according to claim 5, wherein:
one side of the elastic structure (207) facing the needle retraction rod is provided with a first blocking part (215), and the needle retraction rod is provided with a first blocking matching part (304);
with the action of an external force, the needle retraction rod is adapted to move in the inner core until the first blocking matching part (304) abuts against the first blocking part (215) and moves across the first blocking part (215), in which case, the lateral limit part (211) enters the avoidance space (302) in the lateral direction; optionally,
in a case that the first blocking matching part (304) moves across the first blocking part (215), a front end face of the needle retraction rod is flush with a bottom end face of the inner core.

7. The blood collection pen according to claim 6, wherein:
the needle retraction rod is provided with a second blocking matching part (305) axially spaced from the first blocking matching part (304) and closer to the front end;
in a case that the front end face of the needle retraction rod is flush with the bottom end face of the inner core, the first blocking part (215) is provided between the first blocking matching part (304) and the second blocking matching part (305) in the axial direction, the first blocking matching part and the second blocking matching part are adapted to provide a moving resistance for an axial movement of the first blocking part, and the first blocking part is adapted to provide a moving resistance for an axial movement of the first blocking matching part and the second blocking matching part; optionally,
the needle retraction rod is provided with an axially extending needle-retraction-rod locating rib (303), and the first blocking matching part (304) is formed by laterally expanding the needle -retraction -rod locating rib; optionally,
the positions of the avoidance space (302) and the needle-retraction-rod locating rib (303) in the circumferential direction of the needle retraction rod differ by 90 degrees; optionally,
the needle retraction rod is provided with an axially extending needle-retraction-rod locating rib (303), and the second blocking matching part (305) is formed by laterally expanding the needle -retraction -rod locating rib; optionally,
at least one of the abutting faces of the second blocking matching part (305) and the first blocking part (215) in the axial direction is an inclined plane or a convex surface.

8. The blood collection pen according to claim 6, wherein:
the first blocking matching part (304) and the first blocking part (215) are adapted to form an interference in an axial movement; optionally,
at least one of the abutting faces of the first blocking matching part (304) and the first blocking part (215) in the axial direction is an inclined plane or a convex surface.

9. The blood collection pen according to claim 5, wherein:
the lateral limit part (211) is adapted to be located within the avoidance space (302); and
the avoidance space is a concave space, and a side wall near the distal end of the concave space is adapted to interfere with the end face near the distal end of the lateral limit part in axial movement; optionally,
at least one of the axial abutting faces of the side wall near a distal end of the concave space and an end face near a distal end of the lateral limit part is an inclined plane or a convex surface.

10. The blood collection pen according to any one of claims 5-9, wherein:
based on the lateral inward movement of the lateral limit part (211) towards the avoidance space (302), the first axial limit matching part (208) is adapted to disengage from the axial abutment against the first axial limit part (513), so that the inner core is adapted to move towards the distal end under the action of an external force, so that the first axial limit matching part is adapted to contact with the inner wall of the guide sleeve; optionally,
the first axial limit matching part (208) is adapted to abut against or contacting with the inner wall of the guide sleeve to keep the interference fit between the lateral limit matching (211) and the avoidance space (302) in the axial direction after the first axial limit matching part (208) is disengaged from the axial abutment against the first axial limit part (513).

11. The blood collection pen according to claim 2, wherein:
the first axial limit matching part (208) and the first axial limit part (513) are adapted to form an interference in an axial movement; optionally,
at least one of the abutting faces of the first axial limit matching part (208) and the first axial limit part (513) in the axial direction is an inclined plane or a convex surface.

12. The blood collection pen according to claim 2, wherein:
in a case that the first axial limit matching part (208) is disengaged from the abutment against the first axial limit part (513) in the axial direction, the movement of the inner core towards the distal end is adapted to form a loaded abutment against the guide sleeve; optionally,
the inner core is provided with an elastic arm (205), the guide sleeve is provided with a first protrusion (515) protruding laterally inward, and a distal end of the first protrusion is provided with a stepped face; and
in a case that the first axial limit matching part (208) is disengaged from the abutment against the first axial limit part (513) in the axial direction, the inner core is adapted to move to the distal end until an elastic arm end part (206) of the elastic arm (205) moves across the first protrusion (515) to abut against the stepped face in the axial direction, thus forming the loaded abutment.

13. The blood collection pen according to claim 4, wherein:
the first axial limit matching part (208) and the lateral limit part (211) are oppositely provided at two sides of the elastic structure in the lateral direction.

14. The blood collection pen according to claim 1, wherein:
the inner core is provided with an axially extending inner core locating rib (203), the inner wall of the guide sleeve is provided with a guide sleeve groove (508), and the guide groove has a groove bottom end face (509); and
the inner core locating rib is adapted to slid in the guide groove until the locating rib abuts against the groove bottom end face.

15. The blood collection pen according to any one of claims 1-14, further comprising:
a tail cap (9);
a tail cap inner core (8) axially movable in the tail cap, wherein the tail cap inner core and the tail cap are circumferentially limited by a guide structure; and
a blocking member (4) axially movable in the tail cap inner core,
wherein:
a distal end of the inner core (209) is provided with an inner core groove, the distal end of the inner core passes through a guide sleeve hole (507) of the guide sleeve and a tail cap inner core hole (806) of the tail cap inner core in the axial direction, and the blocking member is adapted to be clamped in the inner core groove;
in a case that the first axial limit matching part is disengaged from abutment against the first axial limit part in the axial direction, the tail cap is adapted to drive the tail cap inner core under the action of an axial external force, so that the blocking member and the inner core clamped with the blocking member move axially to the distal end, thereby realizing the loading of the blood collection pen; optionally,
the inn core is provided with an inn core locating rib (203), the inner wall of the guide sleeve is provided with a guide sleeve groove (508), and the guide groove is provided with a groove bottom end face (509); and the inner core locating rib is adapted to slid in the guide groove until the inner core locating rib abuts against the groove bottom end face; optionally,
further comprising: a first spring (12) and a second spring (13), wherein the first spring is sleeved on the inner core and provided between an inner core abutting part (216) of the inner core and a guide sleeve abutting face (511) of the guide sleeve; and the second spring is sleeved on the inner core and provided between a tail cap inner core abutting end face (805) of the tail cap inner core and a blocking member abutting face (404) of the blocking member.
